# EUROPEAN PATENT SPECIFICATION

(11) **EP 3 965 738 B1**
(45) Date of publication and mention of the grant of the patent: **06.08.2025**
(21) Application number: 20732313.0
(22) Date of filing: 07.05.2020
(51) Int. Cl.: A61K 9/1271, A61K 9/1277, A61K 31/519, A61K 31/704

(54) **METHOD FOR PRODUCTION OF LIPOSOMES**
VERFAHREN ZUR HERSTELLUNG VON LIPOSOMEN
PROCÉDÉ DE PRODUCTION DE LIPOSOMES

(30) Priority: 07.05.2019 PT 2019115500
(43) Date of publication of application: 16.03.2022
(73) Proprietor: Universidade do Minho, 4704-553 Braga (PT)
(72) Inventor: PEREIRA GUIMARÃES, Diana Isabel, 4600-235 Amarante (PT); DA COSTA NOGUEIRA, Eugénia Sofia, 4755-415 Barcelos (PT); CAVACO-PAULO, Artur Manuel, 4710-057 Braga (PT)
(74) Representative: Patentree
(86) International application number: PCT/IB2020/054346
(87) International publication number: WO 2020/225769

(56) References cited:
- WO-A1-2017/223135
- US-A1- 2016 228 573
- OSELYS RODRIGUEZ JUSTO ET AL: "Analysis of process parameters on the characteristics of liposomes prepared by ethanol injection with a view to process scale-up: Effect of temperature and batch volume", CHEMICAL ENGINEERING RESEARCH AND DESIGN, ELSEVIER, AMSTERDAM, NL, vol. 89, no. 6, 30 September 2010 (2010-09-30), pages 785 - 792, XP028216220, ISSN: 0263-8762, [retrieved on 20101008], DOI: 10.1016/J.CHERD.2010.09.018

## Description

### Technical field

The present disclosure relates to a method for production of liposomes, in order to obtain high encapsulation efficiency of encapsulated agents with a reduced number of production steps.

### Background Art

Liposomes are defined as artificial microscopic vesicles consisting of an aqueous core surrounded by one or more concentric phospholipid layers (lamellas) [1]. Liposomes have gained extensive attention as carriers for a wide range of therapeutic agents because of being both nontoxic and biodegradable, as they are composed of naturally occurring substances [2]. Liposomes show extensive potential applications as they are able to incorporate hydrophilic (in the aqueous compartment), hydrophobic (within lipidic membrane) and amphiphilic substances (lipid aqueous interface) [3]. Moreover, biologically active materials encapsulated into liposomes are protected from immediate dilution or degradation. For all these reasons liposomes are the most popular nanocarrier systems used since their discovery.

The widespread use of liposomes for several purposes has created the need to develop efficient and reproducible preparation methods with the greatest simplicity as possible. There are different methods for preparation of liposomes, with numerous variants. Because of its simplicity, most laboratory use the lipid thin-film hydration method, first described in 1965 [4]. However, the film method tends to be unsuitable for large scale production. Additionally, there are concerns about the use of chlorinated solvents.

The ethanol injection method is an interesting technique for GMP scaling-up liposomes production. It offers several advantages, e.g. simplicity, GMP friendly solvent, fast implementation and reproducibility, as well as the fact that it does not cause lipid degradation or oxidative alterations. The ethanol injection method was first reported in 1973 by Batzri and Korn [5] as one of the first alternatives for the preparation of small unilamellar vesicles (SUVs) without sonication. By the immediate dilution of the ethanol in the aqueous phase, the lipid molecules precipitate and form bilayer planar fragments. Through energy dissipation in the system (by stirring and/or ultrasonication), the fragments of these lipid bilayers tend to decrease the exposure of the hydrophobic parts of their molecules to the aqueous environment, resulting in the curvature of these fragments which take a quasi-spherical structure. In the following years, several studies have investigated the preparation parameters of the ethanol injection technique (lipid concentration and composition, injection velocity, temperature of both phases, stirring rate, etc.) on the resulting liposome's characteristics (size distribution, zeta potential, drug encapsulation efficiency, etc.) [6].

In the classic ethanolic injection method, the ethanolic phase is in minor percentage comparatively to the aqueous phase, usually 5-10%. After ethanol evaporation, the liposomal dispersion is extruded in order to reduce vesicles size. Briefly, classic ethanolic injection method comprises 3 key steps:
1. Injection of lipids (ethanol) in aqueous phase;
2. Extrusion to reduce liposomes size;
3. Remove non-encapsulated agents.

In general, liposomal therapeutic or imaging agents loading is achieved by either passive or active methods:
Passive loading involves dissolution of dried lipid films in aqueous solutions containing the agent of interest. This approach can only be used for water-soluble agents, and the efficiency of loading is often low (smaller than 5%). This method can be used for a wide range of compounds independently of their chemical structure.
Active loading involves the internalization of agents driven by a liposomal transmembrane pH gradient [7]. This process can be extremely efficient (higher than 95%), resulting in high intraliposomal concentrations and minimal wastage of precious chemotherapeutic agents.
This method (active loading) however requires that molecule have a different protonation state at the extreme pHs of the buffers use inside and outside of liposomes. In such manner a given molecule will diffuse the lipid bilayer when two different pHs are set inside and outside the liposome. Thus, a pH gradient is the driving force to translocate and retain the amphiphilic weak bases and acids [8].

It also reported in the literature the active loading approach for a weakly basic amine therapeutic or imaging agents using a transmembrane ammonium sulfate gradient. In this case, the ammonia gradient drives a pH gradient, leading to active transport of the agent into the liposome. The sulfate then acts as a counterion for the ionized agent, causing it to precipitate within the liposome. This strategy has been applied to the production of liposomal doxorubicin in the case of Doxil. Myocet is another example of liposomal doxorubicin that is remotely loaded, although the pH gradient is established with citric acid [9].

In active loading process, after liposomes preparation with the classic ethanolic injection method, the extra-liposomal phase is removed, and then the agent is added to the extra-liposomal phase and the liposomes are incubated to allow the remote loading process to proceed. Briefly, active loading process comprises 5 key steps:
1. Injection of lipids (ethanol) in aqueous phase;
2. Extrusion to reduce liposomes size;
3. Remove the extra-liposomal phase;
4. Incubation of liposomes with agents;
5. Remove non-encapsulated agents.

Document WO/2013/084208 (Paulo A. et al., 2013, Liposomes and its production method) describes a method of liposomal production which is the lipidic film hydration method.

Document US4752425A (Martin F. et al., 1988, High-encapsulation liposome processing method) describes a method for production of liposomes that uses chloroform. The liposomes produced present 1.5 microns or larger, needing extrusion to size reduction (where the originally encapsulated agent is lost).

Document US5549910A (Szoka F. et al., 1994, Preparation of liposome and lipid complex compositions) describes a method to obtain liposomes containing compounds which exhibit poor solubility in water, alcohols, and halogenated hydrocarbon solvents. In this method the lipids are dissolved in an aprotic solvent solution, which may additionally contain a lower alkanol if needed to solubilize them. This method requires extrusion to obtain liposomes with defined size.

Document US20120171280A1 (Zhang Y, 2011, Method of making liposomes, liposome compositions made by the methods, and methods of using the same) describes a method to obtain liposomes where the aqueous solution comprises ethylenediaminetetraacetic acid (EDTA) to encapsulate ascorbic acid or a salt thereof. The liposome composition has a selected mean particle size diameter of about 200-500 nm.

Document US 5316771A (Barenholz, Y. et al., 1994, Method of amphiphatic drug loading in liposomes by ammonium ion gradient) describes active loading of weak amphiphatic drugs into liposomes using transmembrane gradient.

Document US 5939096A (Clerc, S.Y. and Barenholz, 1999, Stably encapsulating a weak acid drug in liposomes, at a high concentration) describes liposomes encapsulated with a weak acid drug at a high concentration. The method employed a proton shuttle mechanism involving the salt of a weak acid to generate a higher inside/lower outside pH gradient.

Document WO201364911 relates to methods and compositions for producing lipid-encapsulated negatively-charged therapeutic polymers, such as nucleic acid, proteins and peptides, which are encapsulated within a lipid layer.

Document WO0105374 relates to methods and compositions for producing lipid-encapsulated charged therapeutic agent particles, after mixture of preformed lipid vesicles, a charged therapeutic agent (with a charge opposite to the lipid) and a destabilizing agent. Document WO2017/223135 discloses a method for producing nanoparticles for encapsulating an active ingredient comprising the steps of (i) preparing an ethanol solution of lipids and (ii) injecting said solution to an aqueous solution.

### General Disclosure of the Invention

The method of the description has the advantage of achieving a small molecule encapsulation efficiency in a targeted liposome equal to or better than previous methods without extra processing steps to produce nanoparticles. Polycharged molecules, namely with negative charges in their structure at neutral pHs (5-8) like methotrexate
and doxorubicin encapsulate better with this method. Methotextrate has high encapsulation rates and doxorubicin with reduced number of steps. (table 2 and 3).

In the proposed alternative method, a higher encapsulation efficiency of the therapeutic or imaging agent is achieved using a pre-concentration method with an ethanol: aqueous phase at similar volume ratio, and the liposomes may be diluted at the end. The novel proposed method presents a reduced number of steps (only 2) which is desirable in an industrial process. These features are congregated for the first time on the same method, differentiating it from those reported in the literature.

The widespread use of liposomes for several purposes has created the need to develop preparation methods which should be efficient, reproducible and with the greatest simplicity possible. Existing methods remain laborious for industrial scale-up and/or achieving low encapsulation efficiency of the agent of interest. In this way, it is imperative the development of a method with a reduced number of steps and that achieves high encapsulation efficiency of the encapsulated agent.

The lipids used to produce the liposomes may be changed or modified to customize the properties of the liposomal surface and membrane layer. There are different classes of lipids, based in their charge: neutral, cationic, and anionic. The addition of organic molecules to the phosphate head group creates a variety of phospholipid species such as phosphatidylethanolamine (PE), phosphatidylserine (PS), phosphatidylglycerol (PG) and phosphatidylcholine (PC). All these lipids could be used in liposomes production.

Unmodified liposomes do not survive long in circulation, as they are removed by macrophages. One of the first attempts to overcome these problems was focused on the manipulation of lipid membrane components in order to modify bilayer fluidity, as example by inclusion of a steroid. In this way, our liposomal formulations may preferentially contain cholesterol (CH), which may vary from a molar ratio of 35-55%, preferably 35-40%. It was demonstrated that incorporation of cholesterol, into liposomes reduces interaction with blood proteins, by causing increased packing of phospholipids in the lipid bilayer.

Furthermore, in a preferential execution was included a synthetic polymer, polyethyleneglycol (PEG) to the liposomes. PEG-containing liposomes showed less binding to blood proteins, reduced RES uptake, and thus prolonged duration of liposomes in the circulatory system. This has extended the blood circulation of conventional liposomes to drug delivery, the conjugated phospholipid DSPE-MPEG was incorporated in lipidic film of these new formulations, in a molar ratio which may vary between 4-12%, preferably 5-10%.

It has also been demonstrated that surface-modified liposomes with gangliosides have a prolonged circulation time in the blood stream compared to non-modified ones. These characteristics are potentially useful for applications of gangliosides in immunotherapies. Several glycolipids have been tested in studies of RES uptake of liposomes after intravenous injection: the glycolipid GM1 (a brain-tissue-derived monosialoganglioside) significantly decreased RES uptake when incorporated on the liposome surface, and the formulation remained in blood circulation for several hours.

Active targeting exploits specific modification of liposomal surface with a targeting ligand, which can lead to their accumulation at the target site or intracellular delivery to target cells. The inclusion of certain ligands in liposomes allows the release of their contents intracellularly by receptor-mediated endocytosis. Targeting agent integration at membrane surface could be achieved by conjugation to phospholipid or fatty acyl chains or incorporated in the lipidic membrane.

There are different methods for preparation of liposomes, with numerous variants. In the ethanol injection method (5% ethanol), a fraction of the aqueous solution with water-soluble substances is passively encapsulated inside the vesicles. The advantage of this method is its simplicity, but only a very small percentage of water-soluble therapeutic or imaging agents can be encapsulated in this way.

In the remote loading, empty liposomes are generally prepared in an initial salt or low pH buffer. The extra-liposomal phase is then removed using dialysis or size exclusion chromatography, or by titrating the pH to slightly basic conditions. Finally, the agent is added to the extra-liposomal phase and the liposomes are incubated to allow the remote loading process to proceed [6]. The number of steps involved makes the production process difficult to scale up, constituting a barrier to further development of this standard approach.

Hence, we focus our efforts in the optimization of agent encapsulation inside liposomes, with reduced production steps. Indeed, only a low amount of the agent used was encapsulated (e.g. 3-5% to methotrexate drug), being a high amount of agent wasted and this could be an issue in a scale-up process. In order to increase the encapsulated agent numerous conditions were tested in several steps of the production method. Namely: aqueous phase in organic phase containing the phospholipids (instead the opposite), do not remove ethanol, to perform the injection at room temperature instead of at 70 °C, to test different speeds of injection and several concentrations of organic phase (5% until 95%).
The present invention is directed to a method for encapsulating an active ingredient in a liposome consisting of the following sequential steps:
preparing an ethanolic phase by mixing hydrophobic molecules of phospholipids and a steroid with ethanol,
preparing an aqueous phase with an active ingredient and a targeting agent in a buffer solution;
obtaining the liposomes by injecting the ethanolic phase in the aqueous phase, at a temperature from around 50 °C to around 80 °C, wherein the ethanolic/aqueous phase volume ratio is between 1:1 and 3:2;
removing of the ethanol; removing the remaining free active ingredient in a suitable way, namely by tangential flow filtration;
optionally, the step of diluting of the liposomal dispersion 1 to 10-fold in further diluted aqueous phase;
wherein the targeting agent is a peptide that is conjugated with a liposomal component or incorporated in the lipidic membrane;
wherein the ethanol concentration, relative to initial aqueous volume, is between 40% and 60%.

One embodiment of the present invention consists in a new method of production of liposomes,
wherein the hydrophobic components of liposomes are dissolved in ethanol, and injected in an aqueous phase at a rate of approximately 2 - 4 mL/min. under vigorous agitation. The initial volume ratio ethanol: aqueous phase is 1/1. After evaporation of ethanol or tangential flow filtration the liposomal dispersion should be diluted 1 to 10-fold, to the desirable final concentration.

The pre-concentration method comprises 2 key steps:
- Injection of lipids (ethanol) in aqueous phase (1:1 v/v), followed of the dilution;
- Remove non-encapsulated agents.

In an embodiment, this method allows the achievement of high encapsulation efficiencies (e.g. ^{~}40%) for polycharged agent like methotrexate, with a reduced number of steps (only 2). The initial pre-concentration (use of a lower aqueous volume) increases the phospholipid concentration and, consequently allows a higher encapsulation efficiency. Additionally, the use of initial 1:1 of ethanol:aqueous phase volume ratio allows a balance between two phases with different polarities, increasing the encapsulation of the agents.

In another embodiment, the disclosure relates to a method, wherein it further comprises the step of diluting of the liposomal dispersion 1 to 10-fold in further diluted aqueous phase.

In a further embodiment, the disclosure relates to a method, wherein the ethanolic phase is injected at a rate of approximately 2-4 mL/minute.

In a further embodiment, the disclosure relates to a method, wherein the injecting step is performed under agitation.

In a further embodiment, the disclosure relates to a method, wherein the active ingredient is a drug, in particular an anticancer drug, antirheumatic drug, anti-neurodegenerative diseases drug, antioxidant drug, anti-inflammatory drug, antipyretic drug, antibiotic drug, antiviral drug, analgesic drug or combinations thereof.

In another embodiment, the disclosure relates to a method, wherein the targeting agent is a peptide selected from the following list with a degree of identity of at least 90% of the following sequence: SEQ- ID. NO 1, SEQ- ID. NO 2, SEQ- ID. NO 3, or mixtures thereof; comprising at least a sequence 95%, preferably or at least 96% identical, or at least 97% identical, or at least 98% identical, or at least 99% identical, identical to SEQ- ID. NO 1, SEQ- ID. NO 2, SEQ- ID. NO 3, or mixtures thereof.

Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the global (over the whole sequence) alignment of two sequences that maximizes the number of matches and minimizes the number of gaps. The BLAST algorithm (Altschul et al.

(1990) J Mol Biol 215: 403-10) calculates percent sequence identity and performs a statistical analysis of the similarity between the two sequences. The software for performing BLAST analysis is publicly available through the National Centre for Biotechnology Information (NCBI). Global percentages of similarity and identity may also be determined using one of the methods available in the MatGAT software package (Campanella et al., BMC Bioinformatics. 2003 Jul 10; 4:29. MatGAT: an application that generates similarity/identity matrices using protein or DNA sequences). Minor manual editing may be performed to optimise alignment between conserved motifs, as would be apparent to a person skilled in the art. The sequence identity values, which are indicated in the present subject matter as a percentage were determined over the entire amino acid sequence, using BLAST with the default parameters.

The invention relates to a method, wherein the ethanol concentration, relative to the initial aqueous volume, is between 40% and 60%, preferably 50%.

In another embodiment, the disclosure relates to a method, wherein the temperature is 60 °C or 70 °C.

In another embodiment, the disclosure relates to a method, wherein the active ingredient is a polycharged molecule containing at least one negative charge at a pH of around 4 to around 7, particularly methotrexate or doxorubicin.

In a further embodiment, the disclosure relates to a method, wherein the aqueous phase is phosphate buffered saline, PBS.

In another embodiment, the disclosure relates to a method wherein the ethanolic phase comprises anionic, neutral or cationic phospholipids.

In a further embodiment, the disclosure relates to a method, wherein the ethanolic phase comprises phosphatidylcholines, phosphatidylethanolamines, phosphatidylserines, phosphatidylglycerols and/or their derivates or mixtures thereof, in particular 1,2-dioleoyl-sn-glycero-3-phosphoethanolamine.

In another embodiment, the disclosure relates to a method wherein the ethanolic phase comprises a steroid, a stealth agent, a targeting agent, or mixtures thereof. According to the invention, a steroid is present in the ethanolic phase.

In a further embodiment, the disclosure relates to a method wherein the steroid is cholesterol, and/or their derivate, in particular cholesteryl hemisuccinate.

In another embodiment, the disclosure relates to a method wherein the stealth agent is polyethylene glycol, PEG, or gangliosides .

In a further embodiment, the disclosure relates to a method wherein the polyethylene glycol, PEG, is bound to a phospholipid, in particular distearoylphosphatidylethanolamine.

In another embodiment, the disclosure relates to a method wherein the targeting agent is incorporated in the lipidic membrane.

In another embodiment, the disclosure relates to a method wherein the active ingredient is an imaging or therapeutic agent.

In a further embodiment, the disclosure relates to a method wherein the imaging or therapeutic agent is hydrophobic or hydrophilic.

In another further embodiment, the disclosure relates to a method, wherein the imaging agent is a dye.

### Detailed Description

The present disclosure relates to a method for production of liposomes, in order to obtain high encapsulation efficiency of encapsulated agents with a reduced number of production steps, namely avoiding the extrusion step of the classical liposomal production process. The liposomes of the invention are intended to carry a therapeutic agent like an anticancer agent, antioxidant, anti-inflammatory, antipyretic, antibiotic, antiviral, antirheumatic, analgesic, growth-factor, or mixtures thereof.

The method of the description has the advantage of achieving a small molecule encapsulation efficiency in a targeted liposome equal to or better than previous methods without extra processing steps to produce nanoparticles.

In order to increase the encapsulated agent, with reduced production steps, numerous conditions were tested in several steps of the production method. The results demonstrate that the initial percentage of ethanol significantly affected the encapsulation efficiency. A drastic increase in encapsulation efficiency has been noticed as the ethanol volume was higher than the classic 5% (50% relative to the initial aqueous phase), and also using a lower volume of aqueous solution of agent (the sample is afterwards diluted five times to get the usual agent concentration after ethanol evaporation or tangential flow filtration). Vesicles' size has been positively affected by the ethanol volume after this ratio is achieved. Indeed, batches having a higher ethanol volume (>50%) showed larger vesicles (< 150 nm) than liposome batches previously produced. These results may be attributed to the slower diffusion of ethanol related to its volume increase in aqueous phase, leading to the formation of higher sized liposomes due to the slow self-assembly of phospholipids. Accordingly, the smaller the vesicles' size, the smaller the aqueous core volume and the lower obtained encapsulation efficiencies, knowing that the hydrophilic agent is mainly encapsulated in the liposome aqueous core [10, 11]. However, a compromise between the encapsulation efficiency and size was obtained using 50% of initial ethanol volume. Liposomes obtained with this percentage of ethanol present small size (<150 nm) and PDI values (<0.1). Moreover, with these conditions, the extrusion process usually needed to decrease, and uniform vesicles' size is perfectly expendable.

### Production of liposomes

In an embodiment, liposomes composed of DOPE/Cholesterol/DSPE-MPEG (54:36:10, molar ratio) were prepared using the ethanolic injection method. Briefly, lipids (DOPE, cholesterol and DSPE-MPEG) were dissolved in ethanol (5% in the classic ethanol injection method; 50% in the new proposed pre-concentration method, relative to the initial 20% aqueous phase) at 60 °C.

The solution was injected under stirring to an aqueous solution (phosphate buffered saline, PBS). This process is done at 70 °C, remaining during the necessary time to evaporate all the ethanol volume.

In the classic ethanolic injection method liposomes are extruded to reduce their size. In the pre-concentration method, after ethanol evaporation or tangential flow filtration, liposomal dispersion is diluted five times in PBS (remaining 80% of volume is added). The free therapeutic or imaging agent that was not incorporated into liposomes was removed from the samples after passage through a gel filtration chromatography column (GE Healthcare) with 5 kDa cut-off (PD-10 Desalting Columns containing 8.3 mL of Sephadex G-25 Medium). Hydrophilic therapeutic or imaging agents (e.g. methotrexate and doxorubicin) are present in this aqueous phase in the classic ethanol injection method and in the new proposed pre-concentration method. In remote/active loading, after production in ammonium sulfate (120 mM, pH=8.5), the buffer is changed to Trizma^{®}Base sucrose (10%, w/v, buffered at pH 9.0) and empty liposomes are incubated with the therapeutic or imaging agents.

Characterization of liposomes encapsulating methotrexate: comparative study between several production methods.

| | **Z. average (d.nm)** | **PDI** | **Encapsulation efficiency (%)** | **Number of steps** |
|---|---|---|---|---|
| Pre-concentration method (50% ethanol relative to the initial aqueous buffer) | 103.1 ± 2.333 | 0.117 ± 0.003 | 43.4 | 2 (no extrusion) |
| 66% ethanol | 343.3 ± 9.136 | 0.067 ± 0.029 | 41.6 | 2 (no extrusion) |
| 66% ethanol | 124.7 ± 0.635 | 0.061 ± 0.009 | 8.3 | 3 (extrusion) |
| 33% ethanol | 99.66 ± 0.095 | 0.240 ± 0.006 | 7.6 | 3 (extrusion) |
| Classic ethanolic injection method (5% ethanol) | 117.7 ± 1.779 | 0.053 ± 0.018 | 5.7 | 3 (extrusion) |

**Table II - Characterization of liposomes encapsulating doxorubicin: comparative study between several production methods.**

| | **Z. average (d.nm)** | **PDI** | **Encapsulation efficiency (%)** | **Number of steps** |
|---|---|---|---|---|
| Pre-concentration method (50% ethanol relative to the initial 20% aqueous buffer) | 131.2 ± 1.124 | 0.109 ± 0.010 | 84.9 | 2 (no extrusion) |
| Classic ethanolic injection method (5% ethanol) | 115.1 ± 0.551 | 0.048 ± 0.027 | 85.1 | 3 (extrusion) |
| Remote/active loading | 124.8 ± **1.825** | 0.132 ± 0.020 | 76.1 | 5 (extrusion) |

**Table III - A to Z list of cancer drugs including combination treatments**

| **A** | **L** |
|---|---|
| Abemaciclib | Lanreotide Acetate |
| Abiraterone Acetate | Lapatinib Ditosylate |
| Abraxane (Paclitaxel Albumin-stabilized Nanoparticle Formulation) | Lartruvo (Olaratumab) |
| ABVD | Lenalidomide |
| ABVE | Lenvatinib Mesylate |
| ABVE-PC | Lenvima (Lenvatinib Mesylate) |
| AC | Letrozole |
| Acalabrutinib | Leucovorin Calcium |
| AC-T | Leukeran (Chlorambucil) |
| Actemra (Tocilizumab) | Leuprolide Acetate |
| Adcetris (Brentuximab Vedotin) | Levulan Kerastik (Aminolevulinic Acid) |
| ADE | Libtayo (Cemiplimab-rwlc) |
| Ado-Trastuzumab Emtansine | LipoDox (Doxorubicin Hydrochloride Liposome) |
| Adriamycin (Doxorubicin Hydrochloride) | Lomustine |
| Afatinib Dimaleate | Lonsurf (Trifluridine and Tipiracil Hydrochloride) |
| Afinitor (Everolimus) | Lupron (Leuprolide Acetate) |
| Akynzeo (Netupitant and Palonosetron Hydrochloride) | Lupron Depot (Leuprolide Acetate) |
| Aldara (Imiquimod) | Lutathera (Lutetium Lu 177-Dotatate) |
| Aldesleukin | Lutetium (Lu 177-Dotatate) |
| Alecensa (Alectinib) | Lynparza (Olaparib) |
| Alectinib | |
| Alemtuzumab | **M** |
| Alimta (Pemetrexed Disodium) | Marqibo (Vincristine Sulfate Liposome) |
| Aliqopa (Copanlisib Hydrochloride) | Matulane (Procarbazine Hydrochloride) |
| Alkeran for Injection (Melphalan Hydrochloride) | Mechlorethamine Hydrochloride |
| Alkeran Tablets (Melphalan) | Megestrol Acetate |
| Aloxi (Palonosetron Hydrochloride) | Mekinist (Trametinib) |
| Alunbrig (Brigatinib) | Mektovi (Binimetinib) |
| Ameluz (Aminolevulinic Acid) | Melphalan |
| Amifostine | Melphalan Hydrochloride |
| Aminolevulinic Acid | Mercaptopurine |
| Anastrozole | Mesna |
| Apaluta mide | Mesnex (Mesna) |
| Aprepitant | Methotrexate |
| Aranesp (Darbepoetin Alfa) | Methylnaltrexone Bromide |
| Aredia (Pamidronate Disodium) | Midostaurin |
| Arimidex (Anastrozole) | Mitomycin C |
| Aromasin (Exemestane) | Mitoxantrone Hydrochloride |
| Arranon (Nelarabine) | Mogamulizumab-kpkc |
| Arsenic Trioxide | Mozobil (Plerixafor) |
| Arzerra (Ofatumumab) | Mustargen (Mechlorethamine Hydrochloride) |
| Asparaginase Erwinia chrysanthemi | MVAC |
| Atezolizumab | Myleran (Busulfan) |
| Avastin (Bevacizumab) | Mylotarg (Gemtuzumab Ozogamicin) |
| Avelumab | |
| Axicabtagene Ciloleucel | **N** |
| Axitinib | Nanoparticle Paclitaxel (Paclitaxel Albumin-stabilized Nanoparticle Formulation) |
| Azacitidine | Navelbine (Vinorelbine Tartrate) |
| Azedra (Iobenguane 1131) | Necitumumab |
| | Nelarabine |
| **B** | Neratinib Maleate |
| Bavencio (Avelumab) | Nerlynx (Neratinib Maleate) |
| BEACOPP | Netupitant and Palonosetron Hydrochloride |
| Beleodaq (Belinostat) | Neulasta (Pegfilgrastim) |
| Belinostat | Neupogen (Filgrastim) |
| Bendamustine Hydrochloride | Nexavar (Sorafenib Tosylate) |
| Bendeka (Bendamustine Hydrochloride) | Nilandron (Nilutamide) |
| BEP | Nilotinib |
| Besponsa (Inotuzumab Ozogamicin) | Nilutamide |
| Bevacizumab | Ninlaro (Ixazomib Citrate) |
| Bexarotene | Niraparib Tosylate Monohydrate |
| Bicalutamide | Nivolumab |
| BiCNU (Carmustine) | Nplate (Romiplostim) |
| Binimetinib | |
| Bleomycin | **O** |
| Blinaturnomab | Obinutuzumab |
| Blincyto (Blinatumomab) | Odomzo (Sonidegib) |
| Bortezomib | OEPA |
| Bosulif (Bosutinib) | Ofatumumab |
| Bosutinib | OFF |
| Braftovi (Encorafenib) | Olaparib |
| Brentuximab Vedotin | Olaratumab |
| Brigatinib | Omacetaxine Mepesuccinate |
| BuMel | Oncaspar (Pegaspargase) |
| Busulfan | Ondansetron Hydrochloride |
| Busulfex (Busulfan) | Onivyde (Irinotecan Hydrochloride Liposome) |
| | Ontak (Denileukin Diftitox) |
| **C** | Opdivo (Nivolumab) |
| Cabazitaxel | OPPA |
| Cabometyx (Cabozantinib-S-Malate) | Osimertinib |
| Cabozantinib-S-Malate | Oxaliplatin |
| CAF | |
| Calquence (Acalabrutinib) | **P** |
| Campath (Alemtuzumab) | Paclitaxel |
| Camptosar (Irinotecan Hydrochloride) | Paclitaxel Albumin-stabilized Nanoparticle Formulation |
| Capecitabine | PAD |
| CAPOX | Palbociclib |
| Carac (Fluorouracil--Topical) | Palifermin |
| Carboplatin | Palonosetron Hydrochloride |
| CARBOPLATIN-TAXOL | Palonosetron Hydrochloride and Netupitant |
| Carfilzomib | Pamidronate Disodium |
| Carmustine | Panitumumab |
| Carmustine Implant | Panobinostat |
| Casodex (Bicalutamide) | Pazopanib Hydrochloride |
| CEM | PCV |
| Cemiplimab-rwlc | PEB |
| Ceritinib | Pegaspargase |
| Cerubidine (Daunorubicin Hydrochloride) | Pegfilgrastim |
| Cervarix (Recombinant HPV Bivalent Vaccine) | Peginterferon Alfa-2b |
| Cetuximab | PEG-Intron (Peginterferon Alfa-2b) |
| CEV | Pembrolizumab |
| Chlorambucil | Pemetrexed Disodium |
| CHLORAMBUCIL-PREDNISONE | Perjeta (Pertuzumab) |
| CHOP | Pertuzumab |
| Cisplatin | Plerixafor |
| Cladribine | Pomalidomide |
| Clofarabine | Pomalyst (Pomalidomide) |
| Clolar (Clofarabine) | Ponatinib Hydrochloride |
| CMF | Portrazza (Necitumumab) |
| Cobimetinib | Poteligeo (Mogamulizumab-kpkc) |
| Cometriq (Cabozantinib-S-Malate) | Pralatrexate |
| Copanlisib Hydrochloride | Prednisone |
| COPDAC | Procarbazine Hydrochloride |
| Copiktra (Duvelisib) | Procrit (Epoetin Alfa) |
| COPP | Proleukin (Aldesleukin) |
| COPP-ABV | Prolia (Denosumab) |
| Cosmegen (Dactinomycin) | Promacta (Eltrombopag Olamine) |
| Cotellic (Cobimetinib) | Propranolol Hydrochloride |
| Crizotinib | Provenge (Sipuleucel-T) |
| CVP | Purinethol (Mercaptopurine) |
| Cyclophosphamide | Purixan (Mercaptopurine) |
| Cyramza (Ramucirumab) | |
| Cytarabine | **Q** |
| Cytarabine Liposome | [No Entries] |
| Cytosar-U (Cytarabine) | |
| | **R** |
| **D** | Radium 223 Dichloride |
| Dabrafenib | Raloxifene Hydrochloride |
| Dacarbazine | Ramucirumab |
| Dacogen (Decitabine) | Rasburicase |
| Dacomitinib | R-CHOP |
| Dactinomycin | R-CVP |
| Daratumumab | Recombinant Human Papillomavirus (HPV) Bivalent Vaccine |
| Darbepoetin Alfa | Recombinant Human Papillomavirus (HPV) Nonavalent Vaccine |
| Darzalex (Daratumumab) | Recombinant Human Papillomavirus (HPV) Quadrivalent Vaccine |
| Dasatinib | Recombinant Interferon Alfa-2b |
| Daunorubicin Hydrochloride | Regorafenib |
| Daunorubicin Hydrochloride and Cytarabine Liposome | Relistor (Methylnaltrexone Bromide) |
| Decitabine | R-EPOCH |
| Defibrotide Sodium | Retacrit (Epoetin Alfa) |
| Defitelio (Defibrotide Sodium) | Revlimid (Lenalidomide) |
| Degarelix | Rheumatrex (Methotrexate) |
| Denileukin Diftitox | Ribociclib |
| Denosumab | R-ICE |
| DepoCyt (Cytarabine Liposome) | Rituxan (Rituximab) |
| Dexamethasone | Rituxan Hycela (Rituximab and Hyaluronidase Human) |
| Dexrazoxane Hydrochloride | Rituximab |
| Dinutuximab | Rituximab and Hyaluronidase Human |
| Docetaxel | Rolapitant Hydrochloride |
| Doxil (Doxorubicin Hydrochloride Liposome) | Romidepsin |
| Doxorubicin Hydrochloride | Romiplostim |
| Doxorubicin Hydrochloride Liposome | Rubidomycin (Daunorubicin Hydrochloride) |
| Dox-SL (Doxorubicin Hydrochloride Liposome) | Rubraca (Rucaparib Camsylate) |
| Durvalumab | Rucaparib Camsylate |
| Duvelisib | Ruxolitinib Phosphate |
| | Rydapt (Midostaurin) |
| **E** | |
| Efudex (Fluorouracil--Topical) | **S** |
| Eligard (Leuprolide Acetate) | Sancuso (Granisetron) |
| Elitek (Rasburicase) | Sclerosol Intrapleural Aerosol (Talc) |
| Ellence (Epirubicin Hydrochloride) | Siltuximab |
| Elotuzumab | Sipuleucel-T |
| Eloxatin (Oxaliplatin) | Somatuline Depot (Lanreotide Acetate) |
| Eltrombopag Olamine | Sonidegib |
| Emend (Aprepitant) | Sorafenib Tosylate |
| Empliciti (Elotuzumab) | Sprycel (Dasatinib) |
| Enasidenib Mesylate | STANFORD V |
| Encorafenib | Sterile Talc Powder (Talc) |
| Enzalutamide | Steritalc (Talc) |
| Epirubicin Hydrochloride | Stivarga (Regorafenib) |
| EPOCH | Sunitinib Malate |
| Epoetin Alfa | Sustol (Granisetron) |
| Epogen (Epoetin Alfa) | Sutent (Sunitinib Malate) |
| Erbitux (Cetuximab) | Sylatron (Peginterferon Alfa-2b) |
| Eribulin Mesylate | Sylvant (Siltuximab) |
| Erivedge (Vismodegib) | Synribo (Omacetaxine Mepesuccinate) |
| Erleada (Apalutamide) | |
| Erlotinib Hydrochloride | **T** |
| Erwinaze (Asparaginase Erwinia chrysanthemi) | Tabloid (Thioguanine) |
| Ethyol (Amifostine) | TAC |
| Etopophos (Etoposide Phosphate) | Tafinlar (Dabrafenib) |
| Etoposide | Tagrisso (Osimertinib) |
| Etoposide Phosphate | Talc |
| Evacet (Doxorubicin Hydrochloride Liposome) | Talimogene Laherparepvec |
| Everolimus | Tamoxifen Citrate |
| Evista (Raloxifene Hydrochloride) | Tarabine PFS (Cytarabine) |
| Evomela (Melphalan Hydrochloride) | Tarceva (Erlotinib Hydrochloride) |
| Exemestane | Targretin (Bexarotene) |
| | Tasigna (Nilotinib) |
| **F** | Tavalisse (Fostamatinib Disodium) |
| 5-FU (Fluorouracil Injection) | Taxol (Paclitaxel) |
| 5-FU (Fluorouracil--Topical) | Taxotere (Docetaxel) |
| Fareston (Toremifene) | Tecentriq (Atezolizumab) |
| Farydak (Panobinostat) | Temodar (Temozolomide) |
| Faslodex (Fulvestrant) | Temozolomide |
| FEC | Temsirolimus |
| Femara (Letrozole) | Thalidomide |
| Filgrastim | Thalomid (Thalidomide) |
| Firmagon (Degarelix) | Thioguanine |
| Fludarabine Phosphate | Thiotepa |
| Fluoroplex (Fluorouracil--Topical) | Tibsovo (Ivosidenib) |
| Fluorouracil Injection | Tisagenlecleucel |
| Fluorouracil--Topical | Tocilizumab |
| Flutamide | Tolak (Fluorouracil--Topical) |
| FOLFIRI | Topotecan Hydrochloride |
| FOLFIRI-BEVACIZUMAB | Toremifene |
| FOLFIRI-CETUXIMAB | Torisel (Temsirolimus) |
| FOLFIRINOX | Totect (Dexrazoxane Hydrochloride) |
| FOLFOX | TPF |
| Folotyn (Pralatrexate) | Trabectedin |
| Fostamatinib Disodium | Trametinib |
| FU-LV | Trastuzumab |
| Fulvestrant | Treanda (Bendamustine Hydrochloride) |
| Fusilev (Leucovorin Calcium) | Trexall (Methotrexate) |
| | Trifluridine and Tipiracil Hydrochloride |
| **G** | Trisenox (Arsenic Trioxide) |
| Gardasil (Recombinant HPV Quadrivalent Vaccine) | Tykerb (Lapatinib Ditosylate) |
| Gardasil 9 (Recombinant HPV Nonavalent Vaccine) | |
| Gazyva (Obinutuzumab) | **U** |
| Gefitinib | Unituxin (Dinutuximab) |
| Gemcitabine Hydrochloride | Uridine Triacetate |
| GEMCITABINE-CISPLATIN | |
| GEMCITABINE-OXALIPLATIN | **V** |
| Gemtuzumab Ozogamicin | VAC |
| Gemzar (Gemcitabine Hydrochloride) | Valrubicin |
| Gilotrif (Afatinib Dimaleate) | Valstar (Valrubicin) |
| Gleevec (Imatinib Mesylate) | Vandetanib |
| Gliadel Wafer (Carmustine Implant) | VAMP |
| Glucarpidase | Varubi (Rolapitant Hydrochloride) |
| Goserelin Acetate | Vectibix (Panitumumab) |
| Granisetron | VelP |
| Granisetron Hydrochloride | Velcade (Bortezomib) |
| Granix (Filgrastim) | Vemurafenib |
| | Venclexta (Venetoclax) |
| **H** | Venetoclax |
| Halaven (Eribulin Mesylate) | Verzenio (Abemaciclib) |
| Hemangeol (Propranolol Hydrochloride) | Vidaza (Azacitidine) |
| Herceptin (Trastuzumab) | Vinblastine Sulfate |
| HPV Bivalent Vaccine, Recombinant | Vincristine Sulfate |
| HPV Nonavalent Vaccine, Recombinant | Vincristine Sulfate Liposome |
| HPV Quadrivalent Vaccine, Recombinant | Vinorelbine Tartrate |
| Hycamtin (Topotecan Hydrochloride) | VIP |
| Hydrea (Hydroxyurea) | Vismodegib |
| Hydroxyurea | Vistogard (Uridine Triacetate) |
| Hyper-CVAD | Vizimpro (Dacomitinib) |
| | Voraxaze (Glucarpidase) |
| **I** | Vorinostat |
| Ibrance (Palbociclib) | Votrient (Pazopanib Hydrochloride) |
| Ibritumomab Tiuxetan | Vyxeos (Daunorubicin Hydrochloride and Cytarabine Liposome) |
| Ibrutinib | |
| ICE | **W** |
| Iclusig (Ponatinib Hydrochloride) | [No Entries] |
| Idarubicin Hydrochloride | |
| Idelalisib | **X** |
| Idhifa (Enasidenib Mesylate) | Xalkori (Crizotinib) |
| Ifex (Ifosfamide) | Xeloda (Capecitabine) |
| Ifosfamide | XELIRI |
| IL-2 (Aldesleukin) | XELOX |
| Imatinib Mesylate | Xgeva (Denosumab) |
| Imbruvica (Ibrutinib) | Xofigo (Radium 223 Dichloride) |
| Imfinzi (Durvalumab) | Xtandi (Enzalutamide) |
| Imiquimod | |
| Imlygic (Talimogene Laherparepvec) | **Y** |
| Inlyta (Axitinib) | Yervoy (Ipilimumab) |
| Inotuzumab Ozogamicin | Yescarta (Axicabtagene Ciloleucel) |
| Interferon Alfa-2b, Recombinant | Yondelis (Trabectedin) |
| Interleukin-2 (Aldesleukin) | |
| Intron A (Recombinant Interferon Alfa-2b) | **Z** |
| lobenguane 1131 | Zaltrap (Ziv-Aflibercept) |
| Ipilimurnab | Zarxio (Filgrastim) |
| Iressa (Gefitinib) | Zejula (Niraparib Tosylate Monohydrate) |
| Irinotecan Hydrochloride | Zelboraf (Vemurafeni b) |
| Irinotecan Hydrochloride Liposome | Zevalin (Ibritumomab Tiuxetan) |
| Istodax (Romidepsin) | Zinecard (Dexrazoxane Hydrochloride) |
| Ivosidenib | Ziv-Aflibercept |
| Ixabepilone | Zofran (Ondansetron Hydrochloride) |
| Ixazomib Citrate | Zoladex (Goserelin Acetate) |
| Ixempra (Ixabepilone) | Zoledronic Acid |
| | Zolinza (Vorinostat) |
| **J** | Zometa (Zoledronic Acid) |
| Jakafi (Ruxolitinib Phosphate) | Zydelig (Idelalisib) |
| JEB | Zykadia (Ceritinib) |
| Jevtana (Cabazitaxel) | Zytiga (Abiraterone Acetate) |
| | |
| **K** | |
| Kadcyla (Ado-Trastuzumab Emtansine) | |
| Kepivance (Palifermin) | |
| Keytruda (Pembrolizumab) | |
| Kisqali (Ribociclib) | |
| Kymriah (Tisagenlecleucel) | |
| Kyprolis (Carfilzomib) | |

**Table IV- A to Z list of drugs used in rheumatoid arthritis therapy**

| **A** | **M** |
|---|---|
| Abaloparatide (parathyroid hormone) | Magnacet |
| Abatacept | Maxidone |
| Acetaminophen (children and infants) | Meclofenamate sodium |
| Acetaminophen 325mg | Mediproxen |
| Acetaminophen 500 mg | Medrol |
| Acetaminophen 650 mg | Mefenamic acid |
| Acetaminophen with codeine | Meloxicam |
| Acetylsalicylic acid (aspirin) | Menest |
| Actemra | Menostar |
| Activella | Methadone hydrochloride |
| Actonel | Methadose |
| Adalimumab | Methotrexate |
| Addaprin | Methylprednisolone |
| Advil | Miacalcin |
| Alendronate | Millipred |
| Alendronate with vitamin D | Milnacipran |
| Aleve | Mitigare |
| Allopurinol | Mobic |
| Ambien | Morphine sulfate |
| Ambien CR | Morphine sulfate oral solution |
| Amitriptyline hydrochloride | Morphine sulfate with naltrexone |
| Amrix | Motrin |
| Anacin | Motrin IB |
| Anacin (aspirin free) | MS Contin |
| Anakinra | Mycophenolate mofetil |
| Anaprox | |
| Anaprox DS | **N** |
| Apremilast | Nabumetone |
| Arava | Nalfon |
| Arthrotec | Naprelan |
| Atelvia | Naprosyn |
| Avinza | Naproxen and esomeprazole magnesium |
| Azasan | Naproxen sodium (over-the-counter) |
| Azathioprine | Naproxen sodium (prescription) |
| Azulfidine | Neoral |
| Azulfidine EN-Tabs | Neurontin |
| | Norco |
| **B** | |
| Baricitinib | **O** |
| Baycadron | Olumiant |
| Bayer | Opana |
| Belimumab | Oramorph SR |
| Benlysta | Orapred |
| Betamethasone | Orencia |
| Binosto | Otezla |
| Boniva | Otrexup |
| Brisdelle | Oxaprozin |
| Bufferin | Oxycodone |
| | Oxycodone hydrochloride with acetaminophen |
| **C** | Oxycodone with aspirin |
| Calcitonin (nasal spray) | Oxycodone with ibuprofen |
| Cambia | OxyContin |
| Canakinumab | Oxymorphone hydrochloride |
| Cataflam | |
| Celebrex | **P** |
| Celecoxib | Paroxetine |
| Celestone | Paxil |
| CellCept | PediaCare Fever Reducer/Pain Reliever |
| Cequa (solution) | Pediapred |
| Certolizumab pegol | Pegloticase |
| Cevimeline | Pennsaid |
| Children's Tylenol | Percocet |
| Cimzia | Percodan |
| Climara Pro | Pexeva |
| Clinoril | Pilocarpine |
| Cocet | Piroxicam |
| Cocet Plus | Plaquenil |
| Colchicine | Ponstel |
| Colcrys | Prednisolone |
| Combunox | Prednisone |
| Conjugated estrogens/bazedoxifene | Prednisone Intensol |
| ConZip | Pregabalin |
| Cortef | Prelone |
| Cortisone acetate | Premphase |
| Cosentyx | Prempro |
| Cyclobenzaprine | Primlev |
| Cyclophosphamide | Probenecid |
| Cyclosporine | Probenecid and colchicine |
| Cyclosporine ophthalmic emulsion/solution | Prolia |
| Cymbalta | Prozac |
| | |
| **D** | **R** |
| Daypro | Raloxifene hydrochloride |
| Denosumab | Rasuvo |
| Dexamethasone | Rayos |
| DexPak | Reclast |
| Diclofenac | Remicade |
| Diclofenac potassium | Renflexis (infliximab-abda, biosimilar to Remicade) |
| Diclofenac sodium | Reprexain |
| Diclofenac Sodium liquid/gel | Restasis (emulsion) |
| Diclofenac sodium with misoprostol | Rheumatrex |
| Diflunisal | Risedronate sodium |
| Dolophine | Rituxan |
| Duavee | Rituximab |
| Duexis | Roxicet |
| Duloxetine | Roxicodone |
| Dyspel | Rybix ODT |
| | Ryzolt |
| **E** | |
| EC-Naprosyn | **S** |
| Ecotrin | Salagen |
| Effexor XR | Sandimmune |
| Embeda | Sarafem |
| Enbrel | Sarilumab |
| Endocet | Savella |
| Endodan | secukinumab |
| Estrace | Sertraline |
| Estratab | Simponi, Simponi Aria |
| Estrogens | Stelara |
| Estrogens with progesterone | Sulfasalazine |
| Etanercept | Sulfazine |
| Etodolac | Sulfazine EC |
| Evista | Sulindac |
| Evoxac | |
| Excedrin | **T** |
| | Taltz |
| **F** | Teriparatide (parathyroid hormone) |
| Febuxostat | TH Ibuprofen |
| Feldene | Therafeldamine |
| Fenoprofen calcium | Tivorbex |
| FeverAll | Tocilizumab |
| Fexmid | Tofacitinib (extended release) |
| Flexeril | Tofacitinib (immediate release) |
| Fluoxetine | Tolmetin sodium |
| Flurbiprofen | Tramadol |
| Forteo | Tramadol (extended release) |
| Fortical | Tramadol with acetaminophen |
| Fosamax | Trexall |
| Fosamax Plus D | Tylenol Arthritis Pain |
| | Tylenol Extra Strength |
| **G** | Tylenol Regular Strength |
| Gabapentin | Tylenol with Codeine No.2 |
| Gengraf | Tylenol with Codeine No.3 |
| Genpril | Tylenol with Codeine No.4 |
| Golimumab | Tymlos |
| Gralise | |
| | **U** |
| **H** | Uloric |
| Horizant | Ultracet |
| Humira | Ultram |
| Hycet | Ultram-ER |
| Hydrocet | Ustekinumab |
| Hydrocodone bitartrate | |
| Hydrocodone bitartrate with acetaminophen | **V** |
| Hydrocodone bitartrate with ibuprofen | Venlafaxine |
| Hydrocortisone | Veripred 20 |
| Hydrogesic | Vicodin |
| Hydroxychloroquine sulfate | Vicoprofen |
| Hydroxypropyl cellulose pellets | Vimovo |
| Hysingla ER | Vivlodex |
| | Voltaren |
| **I** | Voltaren XR |
| Ibandronate | |
| Ibuprofen (over-the-counter) | **X** |
| Ibuprofen (prescription) | Xatmep |
| Ibuprofen with famotidine | Xeljanz |
| Ilaris | Xeljanz XR |
| Imuran | Xodol |
| Indocin | Xolox |
| Indomethacin | |
| Infant's Tylenol | **Z** |
| Inflectra (infliximab-dyyb, biosimilar to Remicade) | Zamicet |
| Infliximab | Zipsor |
| Intermezzo | Zohydro ER |
| I-Prin | Zoledronic acid |
| ixekizumab | Zoloft |
| | Zolpidem |
| **K** | Zolvit |
| Kadian | Zometa |
| Ketoprofen | Zorvolex |
| Kevzara | Zurampic |
| Kineret | Zydone |
| Krystexxa | Zyloprim |
| KS Ibuprofen | |
| | |
| **L** | |
| Lacrisert | |
| Leflunomide | |
| lesinurad | |
| Lorcet | |
| Lortab | |
| Lyrica | |

**Table IV - A to Z list of dyes**

| **A** | **G** |
|---|---|
| Acetaldehyde-2,4-dinitrophenylhydrazone analytical standard, for environmental analysis | 2-(β-D-Galactosidoxy)naphthol AS-LC |
| 5-Aceta mido-3-[4-[3-[4-(2,4-di-tert-pentylphenoxy)butylcarbamoyl]-4-hydroxy-1-naphthoxy]phenylazo]-4-hydroxy-2,7-naphthalenedisulfonic acid disodium salt Dye content 90 % | Gallocyanine Dye content 90 % |
| Acetone-2,4-dinitrophenylhydrazone environmental standard, 99% | Gentian violet for microscopy (Bact., Hist.) |
| 3-Acetylnoradamantane technical grade, 85% | Gentian Violet meets USP testing specifications |
| Acid Blue 25 Dye content 45 % | Giemsa stain technical grade, used as a blood stain |
| Acid Blue 29 Dye content 40 % | Giemsa stain certified by the Biological Stain Commission |
| Acid Blue 80 Dye content 40 % | Giemsa Stain, Modified Solution (for the staining (of cellular blood components and blood parasites)) |
| Acid Blue 113 Dye content 50 % | Glutaraldehyde bis(2,4-dinitrophenylhydrazone) analytical standard, for environmental analysis |
| Acid Blue 129 Dye content 25 % | Guinea Green B Dye content 50 % |
| Acid Fuchsin used in tissue staining | **H** |
| Acid Fuchsin calcium salt certified by the Biological Stain Commission, Dye content ≥60 % | H+LC:L[49]Cematein for microscopy (Hist.) |
| Acid Green 25 Dye content ≥60 % | Hematoxylin |
| Acid Orange 8 Dye content 65 % | Hematoxylin certified by the Biological Stain Commission |
| Acid Red 1 Dye content 60 % | 1-Heptyl-4-(4-pyridyl)pyridinium bromide 95% |
| Acid Red 183 Dye content 30 % | 6-Hexadecanoyl-2-(((2-(trimethylammonium)ethyl)methyl) amino)naphthalene chloride solid |
| Acid Violet 7 Dye content 40 % | 4'-Hydroxy-4-biphenylcarboxylic acid 99% |
| Acid Yellow 17 Dye content 60 % | 1-(2-Hydroxyethyl)-1,2,3,4-tetrahydro-2,2,4,7-tetramethylquinoline 97% |
| Acid Yellow 25 Dye content 40 % | 8-Hydroxyjulolidine 97% |
| Acridine 97% | Hydroxy naphthol blue disodium salt ACS reagent |
| Acridine Orange hemi(zinc chloride) salt Dye content 90 % | 2-Hydroxy-1,4-naphthoquinone 97% |
| Acridine Orange hydrochloride hydrate 298% (HPLC) | 2-(4-Hydroxyphenyl)-5-pyrimidinol 90% |
| Acridine Orange hydrochloride solution 10 mg/mL in H2O | 1-(4-Hydroxyphenyl)-2,4,6-triphenylpyridinium hydroxide inner salt hydrate 97% |
| Acridine Orange 10-nonyl bromide | **I** |
| Acriflavine | Immersion oil viscosity 150 cSt (lit.) |
| Acriflavine hydrochloride | Immersion oil viscosity 1,250 cSt (lit.) |
| Adrenochrome | Indigo synthetic, Dye content 95 % |
| ADVASEP^{™}-7 solid | Indigo carmine for microscopy (Bact., Hist.), indicator (pH 11.5-14.0) |
| Alcian Blue solution 1% in 3% acetic acid, pH 2.5 | Indigo carmine certified by the Biological Stain Commission, Dye content 85 % |
| Alcian Blue 8GX powder | Indophenol |
| Alcian Blue 8GX certified by the Biological Stain Commission | Indophenol Blue Dye content 60 % |
| Alcian Blue 8GX for microscopy (Bact., Bot., Hist.) | Indoxyl β-D-galactopyranoside |
| Alcianblue 8GX solution for microscopy, 1% in solution (in 3% acetic acid) | Indulin B practical grade |
| Alcian Blue, pyridine variant Dye content ≥85 % | 1-Iodo-3,5-dinitrobenzene 98% |
| Alexa Fluor 350 | Iodonitrotetrazolium chloride Used in colorimetric assays. |
| Alexa Fluor 405 | 5-Iodosalicylaldehyde 97% |
| Alexa Fluor 488 | IR-797 chloride Dye content 70 % |
| Alexa Fluor 532 | Isopentyl nitrite 96% |
| Alexa Fluor 546 | 4-(4-Isothiocyanatophenylazo)-N,N-dimethylaniline 97% |
| Alexa Fluor 555 | **J** |
| Alexa Fluor 568 | Janus Green B certified by the Biological Stain Commission, Dye content 65 % |
| Alexa Fluor 594 | JC-1 solid |
| Alexa Fluor 647 | Jenner's stain suitable for blood stain |
| Alexa Fluor 680 | Jenner's stain certified by the Biological Stain Commission |
| Alexa Fluor 750 | **K** |
| Alizarin Dye content 97 % | Keratin azure |
| Alizarin Blue Black B | **L** |
| Alizarin Red S certified by the Biological Stain Commission | Lacmoid |
| Alizarin Yellow GG Dye content 50 % | Leishman's stain used as histology stain |
| Allophycocyanin | Leit-Silver for electron microscopy |
| Allura Red AC Dye content 80 % | Leucoberbelin Blue I Dye content 65 % |
| Amaranth Dye content 85-95 % | Leucocrystal Violet |
| p-Amidinophenyl p-(6-amidino-2-indolyl)phenyl ether dihydrochloride | Leucomalachite Green |
| 1-Aminoanthraquinone 97% | Light Green SF Yellowish crystalline |
| 4-Amino-1,1'-azobenzene-3,4'-disulfonic acid monosodium salt Dye content 95 % | Light Green SF Yellowish certified by the Biological Stain Commission |
| 3-Amino-3-deoxydigoxigenin hemisuccinamide, succinimidyl ester | Lissamine^{™} Green B Dye content 60 % |
| N-(4-Amino-2,5-diethoxyphenyl)benzamide | Lithium carbonate puriss. p.a., ACS reagent, reagent (for microscopy), ≥99.0% (T) |
| 2-Amino-5,6-dimethylbenzimidazole 97% | LR white acrylic resins Medium |
| 4-Amino-3,6-disulfo-1,8-naphthalic anhydride dipotassium salt | Lucifer Yellow CH dilithium salt fluorescent stain |
| N-(5-Aminopentyl)biotinamide trifluoroacetate salt solid | Lucifer Yellow VS dilithium salt ^{~}85% |
| 2-(3-Aminophenylsulfonyl)ethanol hydrochloride 97% | Lugol solution for microscopy (Bact., Bot.) |
| 4-Aminophthalonitrile 98% | Lumichrome |
| 8-Aminopyrene-1,3,6-trisulfonic acid trisodium salt ≥96.0% (HPCE), solid | **M** |
| Aniline Blue diammonium salt certified by the Biological Stain Commission | Malachite Green oxalate salt Technical grade |
| Aniline Blue solution 2.5% in 2% acetic acid | Malachite Green oxalate salt certified by the Biological Stain Commission |
| N-[(3-(Anilinomethylene)-2-chloro-1-cyclohexen-1-yl)methylene]aniline monohydrochloride 94% | Malachite Green Carbinol hydrochloride Dye content 85 % |
| 8-Anilino-1-naphthalenesulfonic acid | Malonaldehyde bis(phenylimine) monohydrochloride 97% |
| 8-Anilino-1-naphthalenesulfonic acid hemimagnesium salt hydrate for fluorescence, ≥95% (perchloric acid titration) | Martius Yellow Dye content 85 % |
| Anisaldehyde solution | Martius Yellow sodium salt monohydrate 98% |
| Anthrone ACS reagent, 97% | May-Grünwald Stain |
| Arsenazo III calcium-sensitive dye | Melanin from Sepia officinalis |
| Astrazon Orange G | Metanil Yellow for microscopy (Hist.), indicator (pH 1.2-2.3) |
| Auramine O Dye content ≥80 %, certified by the Biological Stain Commission | Metanil Yellow Dye content 70 % |
| 1-Azidoadamantane 97% | 4-(4-Methoxybenzylamino)-7-nitrobenzofurazan |
| Azobenzene 98% | 4-Methoxybiphenyl 97% |
| Azocarmine G | 3-Methoxydiphenylamine 98% |
| Azomethine-H monosodium salt hydrate ^{~}95% | 1-Methoxy-5-methylphenazinium methyl sulfate ≥95% |
| Azophloxine for microscopy (Hist.) | 2-Methoxy-N4-phenyl-1,4-phenylenediamine 95% |
| Azure B certified by the Biological Stain Commission | 6-Methoxy-1,2,3,4-tetrahydro-9H-pyrido[3,4-b]indole 97% |
| Azure B prepared by direct synthesis | 6-Methoxy-1,2,3,4-tetrahydro-9H-pyrido[3,4-b]indole hydrochloride 97% |
| Azure A chloride Dye content ≥70 % | 6-Methoxy-1,2,3,4-tetrahydro-9H-pyrido[3,4-b]indole-1-carboxylic acid 97% |
| Azure A chloride certified by the Biological Stain Commission | 2-Methyl-2-adamantanol 97% |
| Azure A eosinate | 1-(Methylamino)anthraquinone 98% |
| Azure B eosinate | Methyl 3-amino-5,6-dichloro-2-pyrazinecarboxylate 97% |
| Azure II powder | Methyl Blue |
| Azure II eosinate | Methyl Blue certified by the Biological Stain Commission |
| Azure B tetrafluoroborate Dye content 95 % | 4,4'-Methylenebis(N,N-dimethylaniline) 98% |
| **B** | Methylene blue certified by the Biological Stain Commission |
| Bacteriochlorophyll from Rhodopseudomonas sphaeroides | Methylene Blue hydrate suitable for nucleic acid staining, BioReagent |
| Basacryl Red GL Dye content 19 % | Methylene Blue solution for microscopy, concentrate according to Ehrlich, concentrated, aqueous solution |
| Basic Blue 3 Dye content 25 % | Methylene Blue solution 1.4 % (w/v) in 95% ethanol |
| Basic Blue 41 Dye content 40 % | Methylene Blue solution for microbiology |
| Basic Fuchsin for microscopy (Bact., Bot., Hist.), indicator (pH 1.0-3.1) | 4,5-Methylenedioxy-1,2-phenylenediamine dihydrochloride Fluorogenic reagent |
| Basic Fuchsin special for flagella, certified | Methylene Violet (Bernthsen) certified by the Biological Stain Commission, Dye content ≥65 % |
| Basic Fuchsin certified by the Biological Stain Commission, Dye content ≥88 % | Methylene Violet 3RAX Dye content 90 % |
| Basic Fuchsin Dye content >85 % | Methyl Green zinc chloride salt, ^{~}85% |
| Bathocuproinedisulfonic acid disodium salt for spectrophotometric det. of Cu, Fe | Methyl Green zinc chloride salt, for microscopy (Bact., Bot., Hist.) |
| Bathophenanthrolinedisulfonic acid disodium salt hydrate ≥95% | Methyl Green zinc chloride salt, certified by the Biological Stain Commission, Dye content 85 % |
| Benzaldehyde-2,4-dinitrophenylhydrazone environmental standard, 99% | Methyl Green zinc chloride salt, <0.5% crystal violet, Dye content 80 % |
| Benzaldehyde tosylhydrazone 98% | 4-Methyl-3-nitrobenzyl chloride 97% |
| Benzidine ≥98.0% (N) | Methyl Orange ACS reagent, Dye content 85 % |
| Benzidine ISOPAC^{®}, ≥98.0% (N) | 4-Methylphthalonitrile 99% |
| Benzidine dihydrochloride ≥99% (titration) | Methyl Purple in H2O |
| Benzophenone imine 95% | Methyl Red ACS reagent, crystalline |
| N-Benzylideneaniline 99% | Methyl Red hydrochloride ACS reagent |
| N-Benzylidenebenzenesulfonamide 97% | Methyl Red sodium salt Crystalline |
| N-Benzylidenebenzylamine contains 100 ppm MEHQ as stabilizer, 99% | Methyl Red sodium salt ACS reagent, Dye content 95 % |
| Biotin-4-Fluorescein | Methyl violet 2B certified by the Biological Stain Commission |
| bisBenzimide H 33258 ≥98% (HPLC and TLC) | Methyl Violet B base Dye content 85 % |
| bisBenzimide H 33342 trihydrochloride ≥98% (HPLC and TLC) | Mordant Blue 9 Dye content 50 % |
| Bis(cyclohexanone)oxaldihydrazone | Mordant Orange 1 Dye content 70 % |
| Bis(1,3-dibutylbarbituric acid) trimethine oxonol ≥95% (HPLC) | Morin hydrate for microscopy (Fl.), for the determination of Al, Be, Zn, Ga, In, Sc, 1-2 mol/mol water |
| N,N'-Bis(2,5-di-tert-butylphenyl)-3,4,9,10-perylenedicarboximide Dye content 97 % | MTT Formazan powder |
| 1,3-Bis[4-(dimethylamino)phenyl]-2,4-dihydroxycyclobutenediylium dihydroxide, bis(inner salt) Dye content 90 % | Murexide ACS reagent |
| [4-[Bis(2-hydroxyethyl)amino]phenyl]-1,1,2-ethyl en etricarbonitrile 98% | **N** |
| Bismarck Brown R for microscopy (Bact., Hist.) | 2,3-Naphthalenedicarbonitrile 97% |
| Bismarck Brown Y certified by the Biological Stain Commission, Dye content 50 % | 2,3-Naphthalenedicarboximide 97% |
| N,N'-Bis(salicylidene)-1,2-phenylenediamine 97% | α-Naphtholbenzein indicator (pH 8.2-10.0) |
| Brilliant Black BN Dye content 60 % | α-Naphtholbenzein indicator grade |
| Brilliant Blue G solution Concentrate | Naphthol Blue Black Dye content ^{~}50 % |
| Brilliant Blue R Dye content ^{~}50 %, Technical grade | Naphthol Blue Black Dye content 80 % |
| Brilliant Blue R pure | Naphthol Green B Technical grade |
| Brilliant Blue G pure | Naphthol Green B for microscopy (Hist.), for complexometry |
| Brilliant Blue R Concentrate suitable for SDS-PAGE, methanol solution | Naphthol AS-GR phosphate disodium salt |
| Brilliant Blue R Staining Solution ethanol solution | Naphthol AS-MX phosphate disodium salt phosphatase substrate |
| 'Brilliant Cresyl blue' for microscopy (Vit.), mixture of toluidine blue and waterblue | Naphthol AS phosphate |
| Brilliant Cresyl Blue ALD Certified by the Biological Stain Commission | Naphthol AS phosphate disodium salt |
| Brilliant Cresyl Blue ALD certified by the Biological Stain Commission | α-Naphtholphthalein practical grade |
| Brilliant Green Dye content ^{~}90 % | Naphthol Yellow S for microscopy (Hist.), for the precipitation (of amino acids and peptides) |
| Brilliant Green certified by the Biological Stain Commission | 1-Naphthyl red hydrochloride Dye content 85 % |
| Brilliant Yellow Dye content ≥50 % | Neutral Red powder, BioReagent, suitable for cell culture |
| Bromaminic acid sodium salt | Neutral Red Dye content ≥90 % |
| Bromobimane ≥97% | Neutral Red certified by the Biological Stain Commission |
| 4'-Bromo-(1,1'-biphenyl)-4-ol 97% | New Coccine Dye content 75 % |
| Bromochlorophenol Blue Dye content 95 % | Nigrosin certified by the Biological Stain Commission |
| Bromocresol Green ACS reagent, Dye content 95 % | Nigrosin water soluble For use as a biological stain |
| Bromocresol Green sodium salt crystalline | Nile Blue A Dye content ≥75 % |
| Bromocresol Green sodium salt ACS reagent, Dye content 90 % | Nile Blue A certified by the Biological Stain Commission |
| Bromocresol Green sodium salt solution 0.04 wt. % in H2O | Nile Red Technical grade |
| Bromocresol Green/Methyl Red, mixed indicator solution in methanol | Nitrazine Yellow indicator grade, Dye content 85 % |
| Bromocresol Green Sultone Form for microscopy (Bot., Hist., Vit.), indicator (pH 3.8-5.4) | 4-Nitroazobenzene technical grade, 90% |
| Bromocresol Purple BioReagent, suitable for indicator, Dye content 90 % | 3-Nitrobenzaldoxime 99% |
| Bromocresol Purple Technical grade | 4-Nitrobenzenediazonium tetrafluoroborate 97% |
| Bromocresol Purple for microscopy (Hist., Vit.), indicator (pH 5.2-6.8) | 4-Nitroguaiacol 97% |
| Bromocresol Purple sodium salt indicator grade, Dye content 90% | 4-Nitroguaiacol potassium salt hydrate 98% |
| Bromocresol Purple solution 0.04 wt. % in H2O | 4-(4-Nitrophenylazo)resorcinol Dye content 90 % |
| Bromophenol Blue | 2-(3-Nitrophenylsulfonyl)ethanol 97% |
| Bromophenol Blue ACS reagent | 4-Nitrophthalamide 99% |
| Bromophenol Blue sodium salt for molecular biology, for electrophoresis | 3-Nitrophthalimide 97% |
| Bromophenol Blue sodium salt | 4-Nitrophthalimide 98% |
| Bromophenol Blue sodium salt Dye content 90 %, ACS reagent | 3-Nitrophthalonitrile 99% |
| Bromophenol Blue solution 0.04 wt. % in H2O | 4-Nitrophthalonitrile 99% |
| 2-(5-Bromo-2-pyridylazo)-5-(diethylamino)phenol 97% | Nitrotetrazolium Blue chloride ^{~}98% (TLC) |
| Bromothymol Blue ACS reagent, Dye content 95 % | Nitrotetrazolium Blue chloride powder, electrophoresis grade |
| Bromothymol Blue sodium salt powder | Nitrotetrazolium Blue chloride monohydrate |
| Bromothymol Blue sodium salt ACS reagent | 3-Noradamantanamine hydrochloride 95% |
| Bromothymol Blue sodium salt solution 0.04 wt. % in H2O | 3-Noradamantanecarboxylic acid 97% |
| 2-Bromo-1,1,3-trimethoxypropane 95% | Nuclear Fast Red |
| Bromoxylenol Blue indicator grade, Dye content 95 % | Nuclear Fast Red for microscopy (Bot., Hist.) |
| 6-tert-Butyl-2,3-naphthalenedicarbonitrile 94% | Nuclear Fast Red |
| 4-tert-Butylphthalic anhydride 95% | **O** |
| 4-tert-Butylphthalonitrile 98% | Octyl acetate ≥99% |
| N-tert-Butyl-α-(2-sulfophenyl)nitrone sodium salt 95% | Oil Blue N Dye content 96 % |
| Sulfobromophthalein disodium salt hydrate used to study hepatocyte transport functions | Oil Red O certified by the Biological Stain Commission |
| **C** | Oil Red O solution 0.5% in isopropanol |
| Calcein Used for the fluorometric determination of calcium and EDTA titration of calcium in the presence of magnesium. | Oil Red O solution 0.5% in propylene glycol |
| Calcein AM solution 4 mM in DMSO, ≥90% (HPLC), solution | Oil Red EGN |
| Calcium lonophore A23187 mixed calcium magnesium salt Approximate 1:1 molar ratio Ca:Mg. Actual content given on label. | Orange G for NA electrophoresis |
| Calconcarboxylic acid | Orange G certified by the Biological Stain Commission |
| Calmagite indicator grade | Orange G certified by the Biological Stain Commission, Dye content 80 % |
| Calmagite triethanolammonium salt | Orange II sodium salt (Certified by the Biological Stain Commission), Dye content ≥85 % |
| Canada balsam Mounting medium for microscopy | Orange OT Dye content 75 % |
| Carbazole ≥95% (GC) | Orcein synthetic |
| Carbol Fuchsin | Orcein synthetic, certified by the Biological Stain Commission |
| Carbostyril 124 99% | Orcinol monohydrate colorimetric detection reagent |
| 6-Carboxy-4',5'-dichloro-2',7'-dimethoxyfluorescein N-hydroxysuccinimide ester | Osmium tetroxide Sealed ampule. |
| 5-Carboxyfluorescein 99% (HPLC) | Oxacillin sodium salt monohydrate |
| 6-Carboxyfluorescein ^{~}97% (HPLC) | **P** |
| 5-Carboxyfluorescein diacetate ^{~}95% (HPLC) | Para Red Dye content 95 % |
| 5(6)-Carboxyfluorescein diacetate Mixed isomers ≥90% (HPLC) | Pararosaniline hydrochloride |
| N-Carboxymethyl-6-(2,2-dicyanovinyl)-1,2,3,4-tetrahydroquinoline ≥98% (HPLC) | Pararosaniline acetate Dye content 90 % |
| 5-Carboxytetramethylrhodamine | Pararosaniline Base Dye content 95 % |
| Cardiogreen polymethine dye | Pararosaniline Base crystalline |
| Carmine powder | Patent Blue VF Dye content 50 % |
| Carmine certified by the Biological Stain Commission | PDAM for HPLC derivatization |
| Carminic acid | 4-Pentylbicyclo[2.2.2]octane-1-carboxylic acid 99% |
| Celestine blue Dye content 80 % | Perylene sublimed grade, ≥99.5% |
| Chicago Sky Blue 6B powder | Perylene ≥99% |
| Chlorazol Black | Phenazine ethosulfate ≥95% |
| 1-Chloro-9,10-bis(phenylethynyl)anthracene 99% | Phenolphthalein puriss., meets analytical specification of Ph Eur., BP, 98-101% (calc. to the dried substance) |
| 2-Chloro-5-methylaniline 99% | Phenolphthalein ACS reagent |
| 2'-Chloro-5'-methylbenzanilide 97% | Phenolphthalein solution 0.5 wt. % in ethanol: water (1:1) |
| 5-Chloro-2-methylindole 97% | Phenolphthalein bisphosphate tetrasodium salt ^{~}95% |
| 2-Chloro-6-nitrobenzaldehyde 97% | Phenol Red powder, BioReagent, suitable for cell culture |
| Chlorophenol Red indicator grade | Phenol Red ACS reagent |
| Chlorophyll a from Anacystis nidulans algae | Phenol Red sodium salt powder, BioReagent, suitable for cell culture, suitable for insect cell culture |
| Chlorophyll b from spinach ≥90% (HPLC), ≤0.5% Chlorophyll a | Phenol Red sodium salt |
| 4-Chloro-7-sulfobenzofurazan ammonium salt | Phenol Red sodium salt ACS reagent, Dye content 90 % |
| Chromeazurol B | Phenol red solution 0.5%, liquid, sterile-filtered, BioReagent, suitable for cell culture |
| Chromeazurol S Dye content 50 % | Phenosafranin Dye content 80 % |
| Chromotrope 2R suitable for modified Gomori Trichrome stain | 3-Phenoxyphthalonitrile 98% |
| Chromotrope FB Dye content 50 % | 4-Phenoxyphthalonitrile 98% |
| Chrysin 97% | N-[5-(Phenylamino)-2,4-pentadienylidene]aniline monohydrochloride 98% |
| Chrysoidine G for microscopy (Bact., Bot., Vit.) | 4-(Phenylazo)diphenylamine 97% |
| Chrysophenine Dye content 65 % | 4-Phenylazophenol 98% |
| Cibacron Brilliant Yellow 3G-P | 1-Phenyl-2,3-naphthalenedicarboxylic anhydride 98% |
| Clobetasone butyrate ≥98% | 5-Phenyl-2-[2-[[5-phenyl-3-(3-sulfopropyl)-2(3H)-benzoxazolylidene]methyl]-1-butenyl]-3-(3-sulfopropyl)benzoxazolium hydroxide inner salt, sodium salt Dye content 90 % |
| Collodion solution for microscopy, 2% in amyl acetate | 2-(Phenylsulfonyl)ethanol 97% |
| Congo Red certified by the Biological Stain Commission, BioXtra | Phloroglucinol Used to detect the presence of wood fiber. |
| Congo Red Dye content ≥35 % | Phloxine B antibacterial fluorescent dye |
| Coomassie Violet R200 Dye content 50 % | Phloxine B Dye content ≥80 %, certified by the Biological Stain Commission |
| Coproporphyrin I tetramethyl ester ≥90% (HPLC) | Pinacyanol bromide Dye content 95 % |
| Coumarin 6 98% | Pinacyanol chloride |
| Coumarin 7 98% | Poly(1-methoxy-4-(O-disperse Red 1))-2,5-phenylenevinylene |
| Coumarin 314 Dye content 97 % | Ponceau S BioReagent, suitable for electrophoresis |
| Coumarin 334 Dye content 99 % | Ponceau S for microscopy (Hist.) |
| Coumarin 343 Dye content 97 % | Ponceau S Dye content 75 % |
| o-Cresolphthalein indicator grade | Ponceau S solution BioReagent, suitable for electrophoresis, 0.1 % (w/v) in 5% acetic acid |
| o-Cresolphthalein Complexone powder | Ponceau BS Dye content ^{~}60 % |
| m-Cresol Purple indicator grade, Dye content 90 % | Ponceau SS Dye content 80 % |
| m-Cresol Purple sodium salt Dye content 90 % | Ponceau Xylidine Dye content ≥60 % |
| Cresol red indicator grade, Dye content 95 % | Potassium indigotetrasulfonate Dye content 85 % |
| Cresol Red sodium salt indicator grade | Potassium indigotrisulfonate ozone-scavenging reagent |
| Cresyl Violet acetate certified by the Biological Stain Commission | Procion^{®} Red MX-5B Dye content 40 % |
| Crocein Scarlet 7B | Proflavine hemisulfate salt hydrate powder |
| Croconic acid 98% | Propidium iodide ≥94.0% (HPLC) |
| Crotonaldehyde-2,4-dinitrophenylhydrazone analytical standard, for environmental analysis | Propidium iodide solution solution (1.0 mg/ml in water) |
| Crystal Ponceau 6R | Propionaldehyde-2,4-dinitrophenylhydrazone analytical standard, for environmental analysis |
| Crystal Violet Dye content ≥90 % | Prussian blue soluble for microscopy |
| Crystal Violet ACS reagent, ≥90.0% anhydrous basis | Purpurin Dye content 90 % |
| Crystal Violet certified by the Biological Stain Commission | 1-Pyrenebutyric acid N-hydroxysuccinimide ester 95% |
| Crystal violet solution 1%, aqueous solution | 3,4-Pyridinedicarbonitrile 96% |
| Crystal Violet lactone Dye content 97 % | 3,4-Pyridinedicarboxamide 98% |
| Curcumin from Curcuma longa (Turmeric), powder | 1-(2-Pyridylazo)-2-naphthol indicator grade |
| N-[3-Cyano-3-[4-(dicyanomethyl)phenyl]-2-propenylidene]-N-ethyl-ethaniminium inner salt | 4-(2-Pyridylazo)resorcinol 96% |
| 9-Cyano-N,N,N'-triethylpyronine-N'-caproic acid N-hydroxysuccinimide ester chloride ≥85% (HPLC) | 4-(2-Pyridylazo)resorcinol monosodium salt hydrate |
| 9-Cyano-N,N,N'-triethylpyronine-N'-caproic acid N4-(maleimidoethyl)piperazide chloride ≥80% (HPLC) | 3-(2-Pyridyl)-5,6-di(2-furyl)-1,2,4-triazine-5',5"-disulfonic acid disodium salt |
| Cyclohexanone 2,4-dinitrophenylhydrazone ≥99% | 3-(2-Pyridyl)-5,6-diphenyl-1,2,4-triazine-4',4"-disulfonic acid sodium salt BioXtra |
| **D** | Pyrocatechol Violet suitable for indicator |
| DAPI, dilactate ≥98% (HPLC) | Pyrogallol Red |
| Darrow Red certified by the Biological Stain Commission, Dye content ≥65 % | Pyronin Y for NA electrophoresis |
| o-Dianisidine dihydrochloride ≥95% | Pyronin B Dye content ≥30 % |
| o-Dianisidine dihydrochloride Suitable for use in glucose determination | Pyronin B certified by the Biological Stain Commission, Dye content 40 % |
| o-Dianisidine dihydrochloride for enzymic, spectrophotometric determination, vial of 5 mg | Pyronin Y for microscopy (Bot., Fl., Hist.) |
| 4,5-Dibromobenzene-1,2-diol 90%, technical grade | Pyronin Y certified by the Biological Stain Commission, Dye content 50 % |
| 4',5'-Dibromofluorescein Dye content 95 % | **Q** |
| 2,5-Dibromo-6-isopropyl-3-methyl-1,4-benzoquinone | Qdot 525 |
| 3,6-Dibutoxy-1,2-benzenedicarbonitrile 97% | Qdot 565 |
| 2,5-Dibutoxy-4-(4-morpholinyl)benzenediazonium tetrafluoroborate Dye content 95 % | Qdot 605 |
| 1,4-Dibutoxy-2,3-naphthalenedicarbonitrile 99% | Qdot 655 |
| 4-(Dibutylamino)benzaldehyde 98% | Qdot 705 |
| 4-(2-(6-(Dibutylamino)-2-naphthalenyl)ethenyl)-1-(3-sulfopropyl)pyridinium hydroxide inner salt ≥95% (HPLC), solid | Qdot 800 |
| N,N-Dibutylaniline 97% | Quinaldine Red Dye content 95 % |
| 2,4-Dichlorobenzenediazonium 1,5-naphthalenedisulfonate hydrate | Quinoline Yellow for microscopy (Hist.), mixture of mono- and disulfonic acid sodium salt |
| 3,6-Dichloro-1,2-benzenedithiol 95% | Quinoline Yellow Dye content 95 % |
| 1,2-Dichloro-4,5-dinitrobenzene 98% | Quinoline Yellow Mixture of the mono- and disulfonic acids of Quinoline Yellow |
| 2',7'-Dichlorofluorescein ^{~}90% (TLC), crystalline | **R** |
| 2',7'-Dichlorofluorescein ACS reagent | Reactive Black 5 Dye content ≥50 % |
| 2,6-Dichloroindophenol sodium salt hydrate suitable for vitamin C determination, BioReagent | Reactive Blue 4 Dye content 35 % |
| 2,6-Dichloroquinone-4-chloroimide | Reactive Green 19 practical grade |
| 6-(2,2-Dicyanovinyl)-N-(2-hydroxyethyl)-1,2,3,4-tetrahydroquinoline | Reactive Orange 16 Dye content ≥70 % |
| trans-4-(Diethylamino)cinnamaldehyde 98% | Reactive Red 120 |
| 7-Diethylamino-3-(4-maleimidophenyl)-4-methylcoumarin ≥95% (HPLC), solid | Reichardt's dye Dye content 90 % |
| 1,1'-Diethyl-2,2'-carbocyanine iodide 97% | Remazol Brilliant Blue R anthraquinone dye |
| 1,1'-Diethyl-2,2'-cyanine iodide 97% | Renin Substrate 1 |
| 1,1'-Diethyl-4,4'-cyanine iodide | Resazurin sodium salt certified by the Biological Stain Commission |
| N,N-Diethyl-p-phenylenediamine oxalate salt | Resorcinol BioXtra, ≥99% |
| Diethyl 3,4-pyridinedicarboxylate 97% | Resorufin Dye content 95 % |
| 3,3'-Diethylthiacyanine iodide Dye content ^{~}97 % | Rhodamine 6G Dye content 99 % |
| Dihydroethidium ≥95% | Rhodamine 6G Dye content ^{~}95 % |
| Dihydrorhodamine 123 ≥95% | Rhodamine 6G perchlorate Dye content 99 % |
| 2',4'-Dihydroxy-3'-methylacetophenone technical grade, 90% | Rhodamine B ≥95% (HPLC) |
| 1,3-Dihydroxynaphthalene ≥99%, crystalline | Rhodamine 110 chloride Dye content ≥88 % |
| 1,4-Dihydroxy-2,3-naphthalenedicarbonitrile 97% | Rhodamine 123 mitochondrial specific fluorescent dye |
| 4,7-Dihydroxy-1,10-phenanthroline Dye content ≥30 % | Rhodamine B base Dye content 97 % |
| 1,3-Diiminobenz[f]isoindoline 95% | Rhodamine B isothiocyanate-Dextran average mol wt ^{~}10,000 |
| 1,3-Diiminoisoindoline 97% | Rhodamine B isothiocyanate mixed isomers |
| 3,3'-Dimethoxybenzidine dihydrochloride technical grade | Rhodanile Blue A complex of Nile Blue and Rhodamine B., Dye content 75 % |
| (2,5-Dimethoxyphenyl)acetyl chloride 99% | RIM-1 ≥90% (HPLC) |
| 2,4-Dimethoxytoluene 99% | Rose bengal Dye content 95 % |
| 4-(Dimethylamino)benzaldehyde suitable for histochemical demonstration of nitro blue tetrazolium reduction in neutrophils | Rose Bengal sodium salt Dye content ≥85 % |
| 4-(Dimethylamino)cinnamaldehyde chromogenic reagent for indoles and flavanols | Rose Bengal diacetate |
| 4-Dimethylamino-2-methylazobenzene | Rose Bengal lactone 95% |
| 2-[4-(Dimethylamino)styryl]-1-ethylpyridinium iodide ≥99% (HPLC), solid | p-Rosolic acid Dye content 85 % |
| 2-[4-(Dimethylamino)styryl]-N-methylbenzoxazolium perchlorate | Rubrene powder |
| N,N-Dimethyl-4,4'-azodianiline 97% | Ruthenium Red Technical grade |
| N,N'-Dimethyl-9,9'-biacridinium dinitrate used as chemiluminescent reagent | Ruthenium Red for microscopy, ≥85% (calc. on dry substance, AT) |
| N,N-Dimethylindoaniline Dye content 97 % | **S** |
| 3,3-Dimethyl-2-methylene-1-phenylindoline | Saffron crude source of crocetin and crocein |
| N,N-Dimethyl-1-naphthylamine ≥98.0% (GC) | Safranin O Dye content ≥85 % |
| N,N-Dimethyl-4-nitrosoaniline 97% | Safranin O certified by the Biological Stain Commission |
| N,N-Dimethyl-p-phenylenediamine dihydrochloride suitable for peroxidase test, ≥99.0% (titration) | Safranin O for microscopy (Bact., Bot., Hist.), indicator (pH 0.3-1.0) |
| 1,4-Dimethylpyridinium iodide 99% | Schiffs fuchsin-sulfite reagent suitable for detection of glycoproteins |
| 1,4-Dimethylpyridinium p-toluenesulfonate 98% | Scopoletin ≥99% |
| 3,5-Dinitroaniline 97% | Silver diethyldithiocarbamate |
| 4,4'-Dinitro-2-biphenylamine 98% | Silver Enhancer Kit |
| 10-(2',4'-Dinitrophenylazo)-9-phenanthrol | Silver enhancer solution A |
| 3,5-Dinitrosalicylic acid used in colorimetric determination of reducing sugars | Silver enhancer solution B |
| 1,1'-Dioctadecyl-4,4'-bipyridinium dibromide 97% | Silver nitrate BioXtra, >99% (titration) |
| 3,3'-Dioctadecyloxacarbocyanine perchlorate | Silver nitrate ReagentPlus^{®}, ≥99.0% (titration) |
| 4-(2-[6-(Dioctylamino)-2-naphthalenyl]ethenyl)-1-(3-sulfopropyl)pyridinium inner salt ≥95% (HPLC), solid | Silver proteinate ^{~}8% Ag basis |
| 1,3-Diphenylacetone p-tosylhydrazone 98% | Solvent Blue 38 practical grade |
| 2-Diphenylacetyl-1,3-indandione-1-hydrazone 98% | Solvent Blue 59 Dye content 98 % |
| 1,2-Diphenylindole 94% | Solvent Green 3 Dye content 95 % |
| N-(Diphenylmethylene)glycine tert-butyl ester 98% | Stains-All ^{~}95% |
| 2,6-Diphenyl-4H-thiopyran-4-one 96% | trans-4-Stilbenemethanol |
| Direct Blue 71 Dye content 50 % | Sudan I Dye content ≥95 % |
| Direct Blue 15 suitable for Histopaque^{®} system, suitable for viability studies of collagenase-treated rat liver cells | Sudan II Dye content 90 % |
| Direct Red 23 Dye content 30 % | Sudan III Technical grade |
| Direct Red 80 Dye content 25 % | Sudan III certified by the Biological Stain Commission, BioXtra |
| Direct Red 81 Dye content 50 % | Sudan IV certified by the Biological Stain Commission, BioXtra |
| Direct yellow 27 | Sudan IV Dye content ≥80 % |
| Disperse Black 9 Dye content 97 % | Sudan Black B certified by the Biological Stain Commission |
| Disperse Blue 1 Dye content 30 % | Sudan Blue II Dye content 98 % |
| Disperse Blue 3 Dye content 20 % | Sudan Orange G Dye content 85 % |
| Disperse Blue 14 Dye content 97 % | Sudan Red 7B for microscopy (Bot., Hist.) |
| Disperse Orange 1 Dye content ^{~}15 % | Sudan Red 7B Dye content 95 % |
| Disperse Orange 13 Dye content 90 % | Sulfanilic acid azochromotrop ≥80% |
| Disperse Orange 13 Dye content 15 % | 2-Sulfobenzoic acid cyclic anhydride technical grade, 90% |
| Disperse Yellow 3 Dye content 30 % | Sulfochlorophenol S sodium calcium salt |
| Dithizone Practical Grade | Sulforhodamine 101 |
| Dithizone ACS reagent, ≥85.0% | Sulforhodamine B sodium salt Technical grade |
| **E** | Sunset Yellow FCF Dye content 90 % |
| Eosin B certified by the Biological Stain Commission, Dye content 90% | SYBR^{®} Green II RNA gel stain 10,000 x in DMSO Green Alternative |
| Eosin B for microscopy (FI., Hist.), adsorption and fluorescent indicator | SYBR^{®} Green I nucleic acid gel stain 10,000 x in DMSO Green Alternative |
| Eosin B Dye content 95 % | SynaptoGreen^{™} C4 ≥95% (HPLC), solid |
| Eosin Y Dye content ^{~}99 % | SynaptoRed^{™} C2 ≥95% (HPLC), solid |
| Eosin Y | Syringaldazine 99% |
| Eosin Y disodium salt Dye content ≥85 % | **T** |
| Eosin Y disodium salt certified by the Biological Stain Commission | Tannic acid Source: Chinese natural gall nuts |
| Eosin Y solution 5 wt. % in H2O | Tetrabromophenol Blue Dye content 85 % |
| Eriochrome^{®} Black T ACS reagent (indicator grade) | Tetrabromophenol Blue sodium salt Dye content 85 % |
| Erioglaucine disodium salt | 3',3",5',5"-Tetrabromophenolphthalein ethyl ester potassium salt indicator grade |
| Erythrosin B Dye content ≥95 % | 3,4,5,6-Tetrabromophenolsulfonephthalein Dye content 95% |
| Erythrosin B certified by the Biological Stain Commission, Dye content 90 % | Tetrabromo-2-sulfobenzoic acid cyclic anhydride |
| Erythrosin extra bluish for microscopy (Bact., Hist.), adsorption and fluorescent indicator | Tetrabutylammonium bis(3,6-dichloro-1,2-benzenedithiolato)nickelate 98% |
| Erythrosin extra bluish certified by the Biological Stain Commission | Tetrabutylammonium bis(4-methyl-1,2-benzenedithiolato)nickelate 98% |
| Erythrosin yellowish blend | Tetrachlorophthalonitrile 98% |
| Ethidium bromide ^{~}95% (HPLC) | Tetrachrome Stain (MacNeal) |
| Ethidium bromide monoazide ≥95% (HPLC), solid | 2,3,6,7-Tetrahydro-8-hydroxy-1H,5H-benzo[ij]quinolizine-9-carboxaldehyde 98% |
| Ethidium homodimer I solution ≥95%, 2 mM in DMSO | 1,2,3,4-Tetrahydro-2,2,4,7-tetramethylquinoline 97% |
| 5-{3-Ethoxy-4-(3-ethyl-5-methyl-2(3H)-benzothiazolylidene)-2-butenylidene]-3-ethyl-2-[(3-ethyl-4,5-diphenyl-2(3H)-thiazolylidene)methyl]-4,5-dihydro-4-oxothiazolium iodide Dye content 95 % | 3',3",5',5"-Tetraiodophenolsulfonephthalein Dye content 90 % |
| 3-Ethoxy-4-methoxybenzaldehyde 99% | 1,2,3,3-Tetramethyl-3H-indolium iodide 98% |
| 2-[3-(3-Ethyl-2(3H)-benzothiazolylidene)-2-methyl-1-propenyl]-3-[3-(sulfooxy)butyl]benzothiazolium hydroxide inner salt Dye content 90 % | 2,2,4,4-Tetramethyl-3-pentanone imine 95% |
| 5-Ethyl-5,6-dihydro-3,8-dinitro-6-phenyl-6-phenanthridinol 97% | N,N,N',N'-Tetramethyl-p-phenylenediamine dihydrochloride ≥95%, powder |
| Ethyl 3,5-dimethyl-2-pyrrolecarboxylate 98% | Tetramethylrhodamine-5-isothiocyanate |
| Ethyl eosin certified by the Biological Stain Commission | Tetramethylrhodamine methyl ester perchlorate ≥95% |
| Ethyl 4'-hydroxy-4-biphenylcarboxylate 98% | Tetranitroblue tetrazolium chloride |
| 3-Ethyl-2-methylbenzothiazolium iodide | Tetrazolium Blue Chloride used in colorimetric determination of reducing compounds |
| Ethyl Orange sodium salt indicator grade, Dye content 90 % | Tetrazolium Violet |
| 2-(Ethylthio)benzothiazole 97% | TFLZn potassium salt ≥90% (TLC) |
| 2-Ethyl-2-thiopseudourea hydrobromide 98% | TFLZn-AM ≥90% (TLC) |
| N-Ethyl-o-toluidine 97% | Thiazole Orange Dye content ^{~}90 % |
| Ethyl Violet cationic triarylmethane dye | Thiazolyl Blue Tetrazolium Bromide 98% |
| Ethyl viologen diperchlorate 98% | Thioflavine S practical grade |
| Evans Blue Dye content ≥75 % | Thioflavin T used as stain for amyloid |
| **F** | Thionin acetate salt certified by the Biological Stain Commission, Dye content ≥85 % |
| Fast Black K Salt hemi(zinc chloride) salt practical grade | Thymol Blue ACS reagent |
| Fast Blue BB Salt hemi(zinc chloride) salt Dye content ≥80 % | Thymol Blue sodium salt ACS reagent, Dye content 95 % |
| Fast Blue BB Salt hemi(zinc chloride) salt for microscopy (Hist.) | Thymolphthalein ACS reagent, Dye content 95 % |
| Fast Blue RR | Thymoquinone ≥98% |
| Fast Blue RR Salt crystalline | o-Tolidine ≥97%, powder |
| Fast Blue B Salt Dye content ^{~}95 % | Toluidine Blue O Technical grade |
| Fast Corinth V zinc chloride double salt Dye content 90 % | Toluidine Blue O certified by the Biological Stain Commission |
| Fast Dark Blue R Salt | Toluidine Red Dye content 70 % |
| Fast Garnet GBC sulfate salt diazonium dye | 3,4,5-Trihydroxybenzamide 98% |
| Fast Garnet GBC base 97% | Tris(4-nitrophenyl)amine technical grade |
| Fast Green FCF Dye content ≥85 % | Trypan Blue Dye content 60 % |
| Fast Green FCF certified by the Biological Stain Commission | Trypan Blue powder, BioReagent, suitable for cell culture |
| Fast Red B tetrafluoroborate salt Dye content 95 % | Trypan Blue solution 0.4%, liquid, sterile-filtered, suitable for cell culture |
| Fast Red ITR | **U** |
| Fast Red RC Salt | Uniblue A sodium salt |
| Fast Red Violet LB base | **V** |
| Fast Red Violet LB Salt Dye content ≥90 % | Valeraldehyde-2,4-dinitrophenylhydrazone environmental standard, 99% |
| Fat Brown B | Vanillin azine 99% |
| Ferroin indicator solution 0.1 wt. % in H2O | Variamine Blue RT Salt |
| FIM-1 | Victoria Blue R Dye content 80 % |
| FIM-1 diacetate | Victoria Pure Blue BO Dye content 90 % |
| Fluorescein sodium salt used as fluorescent tracer | Violamine R Dye content 60 % |
| Fluorescein-5-EX N-hydroxysuccinimide ester | **W** |
| Fluoresceinamine, isomer I | Wright stain suita ble for blood stain |
| Fluorescein diacetate used as cell viability stain | Wright stain certified by the Biological Stain Commission |
| Fluorescein (free acid) Dye content 95 % | Wright Stain solution for microscopy |
| Fluorescein 5(6)-isothiocyanate ≥90% (HPLC) | **X** |
| Fluorescein isothiocyanate isomer I suitable for protein labeling, ≥90% (HPLC), powder | p-Xylene-bis(N-pyridinium bromide) ≥95% (TLC) |
| Fluorescein isothiocyanate isomer I ≥97.5% (HPLC) | Xylene Cyanol FF Dye content ≥75 % |
| Fluorescein isothiocyanate isomer I-Celite^{®} suitable for fluorescent labeling techniques | Xylene Cyanol FF for molecular biology, BioReagent |
| Fluorescein Sodium salt - CAPS solution for fluorescence, ≥95.0% (HPLC) | Xylenol Blue indicator grade, Dye content 90 % |
| Fluorescent Brightener 28 used as a stain and brightening agent | Xylenol Orange tetrasodium salt ACS reagent |
| Fluorinert^{™} FC-40 | Xylidyl blue I |
| 4-Formylbenzene-1,3-disulfonic acid disodium salt hydrate 97% | **Z** |
| Fura 2-AM ≥95% (HPLC) | Zincon sodium salt Dye content ≥75 % |
| Fura-2 LeakRes (AM) ≥85% | Zinquin ≥95% (HPLC), solid |
| Fusidic acid | Zinquin ethyl ester ≥95% (HPLC), solid |
| Plasmocorinth B Dye content 60 % | |

Where ranges are given, endpoints are included. Furthermore, it is to be understood that unless otherwise indicated or otherwise evident from the context and/or the understanding of one of ordinary skill in the art, values that are expressed as ranges can assume any specific value within the stated ranges in different embodiments of the invention, to the tenth of the unit of the lower limit of the range, unless the context clearly dictates otherwise. It is also to be understood that unless otherwise indicated or otherwise evident from the context and/or the understanding of one of ordinary skill in the art, values expressed as ranges can assume any subrange within the given range, wherein the endpoints of the subrange are expressed to the same degree of accuracy as the tenth of the unit of the lower limit of the range.

The term "comprising" whenever used in this document is intended to indicate the presence of stated features, integers, steps, components, but not to preclude the presence or addition of one or more other features, integers, steps, components or groups thereof.

The disclosure is of course not in any way restricted to the embodiments described and a person with ordinary skill in the art will foresee many possibilities to modifications thereof without departing from the disclosure as defined in the appended claims.

The above described embodiments are obviously combinable.

The following dependent claims set out particular embodiments of the disclosure.

### SEQUENCE LISTING

SEQ- ID. NO.1: Folic acid-DRDDQAAWFSQY
SEQ- ID. NO 2: KDEPQRRSARLSAKPAPPKPEPKPKKAPAKK-DRDDQAAWFSQY
SEQ- ID. NO 3: YQSFWAAQDDRD-KDEPQRRSARLSAKPAPPKPEPKPKKAPAKK

### REFERENCES

[1] Wang, A. Z., Langer, R., and Farokhzad, O. C., Nanoparticle Delivery of Cancer Drugs, Annual Review of Medicine. 2012, 63, 185-198.
[2] Hong, M.-S., Lim, S.-J., Lee, M.-K., Kim, Y. B., and Kim, C.-K., Prolonged Blood Circulation of Methotrexate by Modulation of Liposomal Composition, Drug Delivery. 2001, 8, 231-237.
[3] Laouini, A., Jaafar-Maalej, C., Limayem-Blouza, I., Sfar, S., Charcosset, C., and Fessi, H., Preparation, Characterization and Applications of Liposomes: State of the Art, Journal of Colloid Science and Biotechnology. 2012, 1, 147-168.
[4] Bangham, A. D., Standish, M. M., and Watkins, J. C., Diffusion of univalent ions across the lamellae of swollen phospholipids, Journal of Molecular Biology. 1965, 13, 238-IN27.
[5] Batzri, S. and Korn, E. D., Single bilayer liposomes prepared without sonication, Biochimica et Biophysica Acta (BBA) - Biomembranes. 1973, 298, 1015-1019.
[6] Naeff, R., Feasibility of topical liposome drugs produced on an industrial scale, Advanced Drug Delivery Reviews. 1996, 18, 343-347.
[7] Sur, S., Fries, A. C., Kinzler, K. W., Zhou, S., and Vogelstein, B., Remote loading of preencapsulated drugs into stealth liposomes, Proceedings of the National Academy of Sciences of the United States of America. 2014, 111, 2283-2288.
[8] Gubernator, J., Active methods of drug loading into liposomes: Recent strategies for stable drug entrapment and increased in vivo activity. Vol. 8. 2011. 565-80.
[9] Duong, A. D., Collier, M. A., Bachelder, E. M., Wyslouzil, B. E., and Ainslie, K. M., One Step Encapsulation of Small Molecule Drugs in Liposomes via Electrospray-Remote Loading, Mol Pharm. 2016, 13, 92-9.
[10] Jaafar-Maalej, C., Diab, R., Andrieu, V., Elaissari, A., and Fessi, H., Ethanol injection method for hydrophilic and lipophilic drug-loaded liposome preparation, J Liposome Res. 2010, 20, 228-43.
[11] Pons, M., Foradada, M., and Estelrich, J., Liposomes obtained by the ethanol injection method, International Journal of Pharmaceutics. 1993, 95, 51-56.

## Claims

1. Method for encapsulating an active ingredient in a liposome consisting of the following sequential steps:
preparing an ethanolic phase by mixing hydrophobic molecules of phospholipids and a steroid with ethanol,
preparing an aqueous phase with an active ingredient and a targeting agent in a buffer solution;
obtaining the liposomes by injecting the ethanolic phase in the aqueous phase, at a temperature from around 50 °C to around 80 °C, wherein the ethanolic/aqueous phase volume ratio is between 1:1 and 3:2;
removing the ethanol;
removing the remaining free active ingredient in a suitable way, namely by tangencial flow filtration;
optionally, the step of diluting of the liposomal dispersion 1 to 10-fold in further diluted aqueous phase;
wherein the targeting agent is a peptide that is conjugated with a liposomal component or incorporated in the lipidic membrane;
wherein the ethanol concentration, relative to initial aqueous volume, is between 40% and 60%.

2. Method according to the previous claim wherein the ethanol removal is by evaporation or tangential flow filtration.

3. Method according to any of the previous claims, wherein the steroid is cholesterol.

4. Method according to any of the previous claims, wherein the ethanolic phase is injected at a rate of approximately 2-4 mL/minute.

5. Method according to the previous claims, wherein the ethanol concentration, relative to the initial aqueous volume is 50%.

6. Method according to any of the previous claims, wherein the temperature is 60 °C or 70 °C.

7. Method according to any of the previous claims, wherein the active ingredient is a polycharged molecule containing at least one negative charge at a pH of around 4 to around 7.

8. Method according to any of the previous claims, wherein the active ingredient is a drug, an imaging or a therapeutic agent, in particular an anticancer drug, antirheumatic drug, anti-neurodegenerative diseases drug, antioxidant drug, anti-inflammatory drug, antipyretic drug, antibiotic drug, antiviral drug, analgesic drug or combinations thereof, in particular the imaging agent is a dye.

9. Method according to any of the previous claims, wherein the injecting step is performed under agitation.

10. Method according to any of the previous claims, wherein the targeting agent is at least a peptide selected from the following list with a degree of identity of at least 90% of the following sequence: SEQ- ID. NO 1, SEQ- ID. NO 2 , SEQ- ID. NO 3, or mixtures thereof.

11. Method according to any of the previous claims, wherein the aqueous phase is phosphate buffered saline, PBS.

12. Method according to any of the previous claims wherein the ethanolic phase comprises anionic, neutral, or cationic phospholipids, preferably the ethanolic phase phospholipids are selected from a list consisting of: phosphatidylcholines, phosphatidylethanolamines, phosphatidylserines, phosphatidylglycerols and/or mixtures thereof, in particular 1,2-dioleoyl-sn-glycero-3-phosphoethanolamine.

13. Method according to any of the previous claims wherein the ethanolic phase further comprises a stealth agent, a targeting agent, or mixtures thereof.

14. Method according to claim 13 wherein the stealth agent is polyethylene glycol, PEG, or gangliosides, preferably the polyethylene glycol, PEG, is bound to a phospholipid, in particular distearoylphosphatidylethanolamine.

15. Method according to any of the previous claims wherein the targeting agent is incorporated in the lipidic membrane and/or wherein the active ingredient is an imaging or therapeutic agent.

16. Method according to any of the previous claims wherein the active ingredient is an imaging or therapeutic agent which is hydrophobic or hydrophilic; preferably wherein the imaging agent is a dye or wherein the active ingredient is methotrexate, doxorubicin and/or mixtures thereof.

## Patentansprüche

1. Verfahren zur Umhüllung eines Wirkstoffs in einem Liposom, bestehend aus den folgenden aufeinanderfolgenden Schritten:
Herstellen einer ethanolischen Phase durch Mischen hydrophober Phospholipidmoleküle und eines Steroids mit Ethanol,
Herstellen einer wässrigen Phase mit einem Wirkstoff und einem Targeting-Mittel in einer Pufferlösung;
Gewinnen der Liposomen durch Einspritzen der ethanolischen Phase in die wässrige Phase bei einer Temperatur von etwa 50 °C bis etwa 80 °C, wobei das Volumenverhältnis ethanolische/wässrige Phase zwischen 1:1 und 3:2 liegt;
Entfernen des Ethanols;
Entfernen des verbleibenden freien Wirkstoffs in geeigneter Weise, namentlich durch Tangentialstromfiltration;
gegebenenfalls den Schritt des Verdünnens der liposomalen Dispersion um das 1-bis 10-fache in einer weiteren verdünnten wässrigen Phase;
wobei das Targeting-Mittel ein Peptid ist, das mit einer liposomalen Komponente konjugiert oder in der Lipidmembran enthalten ist;
wobei die Ethanolkonzentration, bezogen auf das wässrige Ausgangsvolumen, zwischen 40 % und 60 % liegt.

2. Verfahren nach dem vorangehenden Anspruch, wobei das Ethanol durch Verdampfen oder Tangentialstromfiltration entfernt wird.

3. Verfahren nach einem der vorangehenden Ansprüche, wobei das Steroid Cholesterin ist.

4. Verfahren nach einem der vorangehenden Ansprüche, wobei die ethanolische Phase mit einer Geschwindigkeit von etwa 2-4 mL/Minute injiziert wird.

5. Verfahren nach einem der vorangehenden Ansprüche, wobei die Ethanolkonzentration, bezogen auf das wässrige Ausgangsvolumen, 50 % beträgt.

6. Verfahren nach einem der vorangehenden Ansprüche, wobei die Temperatur 60 °C oder 70 °C beträgt.

7. Verfahren nach einem der vorangehenden Ansprüche, wobei der Wirkstoff ein mehrfach geladenes Molekül ist, das mindestens eine negative Ladung bei einem pH-Wert von etwa 4 bis etwa 7 enthält.

8. Verfahren nach einem der vorangehenden Ansprüche, wobei der Wirkstoff ein Arzneimittel, ein bildgebendes oder ein therapeutisches Mittel ist, insbesondere ein Arzneimittel gegen Krebs, ein Arzneimittel gegen Rheuma, ein Arzneimittel gegen neurodegenerative Erkrankungen, ein antioxidatives Arzneimittel, ein entzündungshemmendes Arzneimittel, ein fiebersenkendes Arzneimittel, ein antibiotisches Arzneimittel, ein antivirales Arzneimittel, ein schmerzlinderndes Arzneimittel oder Kombinationen davon, wobei das bildgebende Mittel insbesondere ein Farbstoff ist.

9. Verfahren nach einem der vorangehenden Ansprüche, wobei der Schritt des Injizierens unter Umrühren erfolgt.

10. Verfahren nach einem der vorangehenden Ansprüche, wobei das Targeting-Mittel mindestens ein aus folgender Liste ausgewähltes Peptid ist und einen Identitätsgrad von mindestens 90 % der folgenden Sequenz aufweist: SEQ- ID. NO 1, SEQ- ID. NO 2, SEQ- ID. NO 3, oder Mischungen davon.

11. Verfahren nach einem der vorangehenden Ansprüche, wobei die wässrige Phase eine phosphatgepufferte Salzlösung, PBS, ist.

12. Verfahren nach einem der vorangehenden Ansprüche, wobei die ethanolische Phase anionische, neutrale oder kationische Phospholipide umfasst, wobei die Phospholipide der ethanolischen Phase bevorzugt aus einer Liste ausgewählt werden, umfassend: Phosphatidylcholine, Phosphatidylethanolamine, Phosphatidylserine, Phosphatidylglycerine und/oder Mischungen davon, insbesondere 1,2-dioleoyl-sn-glycero-3-phosphoethanolamin.

13. Verfahren nach einem der vorangehenden Ansprüche, wobei die ethanolische Phase ferner ein tarnendes Mittel, ein Targeting-Mittel oder Mischungen davon enthält.

14. Verfahren nach Anspruch 13, wobei das tarnende Mittel Polyethylenglykol, PEG, oder Ganglioside ist, wobei das Polyethylenglykol, PEG, bevorzugt an ein Phospholipid, insbesondere Distearoylphosphatidylethanolamin, gebunden ist.

15. Verfahren nach einem der vorangehenden Ansprüche, bei dem das Targeting-Mittel in der Lipidmembran enthalten ist und/oder bei dem der Wirkstoff ein bildgebendes oder therapeutisches Mittel ist.

16. Verfahren nach einem der vorangehenden Ansprüche, wobei der Wirkstoff ein bildgebendes oder therapeutisches Mittel ist, das hydrophob oder hydrophil ist; wobei das bildgebende Mittel bevorzugt ein Farbstoff ist oder wobei der Wirkstoff Methotrexat, Doxorubicin und/oder Mischungen davon ist.

## Revendications

1. Procédé d'encapsulation d'un principe actif dans un liposome consistant en les étapes séquentielles suivantes :
préparer une phase éthanolique en mélangeant des molécules hydrophobes de phospholipides et un stéroïde avec de l'éthanol ;
préparer une phase aqueuse avec un principe actif et un agent de ciblage dans une solution tampon ;
obtenir les liposomes en injectant la phase éthanolique dans la phase aqueuse, à une température allant de environ 50 °C à environ 80 °C, dans lequel le rapport de volume de la phase éthanolique/aqueuse se situe entre 1:1 et 3:2 ;
retirer l'éthanol ;
retirer le principe actif libre restant de manière adaptée, notamment par filtration de flux tangentiel ;
optionnellement, l'étape consistant à diluer la dispersion liposomale de 1 pour 10 dans une phase aqueuse plus diluée ;
dans lequel l'agent de ciblage est un peptide qui est conjugué avec un composant liposomal ou incorporé dans la membrane lipidique ;
dans lequel la concentration en éthanol, par rapport au volume aqueux initial, se situe entre 40% et 60%.

2. Procédé selon la revendication précédente dans lequel le retrait de l'éthanol se fait par évaporation ou filtration de flux tangentiel.

3. Procédé selon l'une quelconque des revendications précédentes, dans lequel le stéroïde est du cholestérol.

4. Procédé selon l'une quelconque des revendications précédentes, dans lequel la phase éthanolique est injectée à un taux d'environ 2-4 mL/minute.

5. Procédé selon les revendications précédentes, dans lequel la concentration en éthanol par rapport au volume aqueux initial est de 50%.

6. Procédé selon l'une quelconque des revendications précédentes, dans lequel la température est de 60 °C ou 70 °C.

7. Procédé selon l'une quelconque des revendications précédentes, dans lequel le principe actif est une molécule polychargée contenant au moins une charge négative à un pH allant de environ 4 à environ 7.

8. Procédé selon l'une quelconque des revendications précédentes, dans lequel le principe actif est un médicament, un agent d'imagerie ou thérapeutique, en particulier un médicament anticancéreux, un médicament antirhumatismal, un médicament contre les maladies neurodégénératives, un médicament antioxydant, un médicament anti-inflammatoire, un médicament antipyrétique, un médicament antibiotique, un médicament antiviral, un médicament analgésique ou des combinaisons de ceux-ci, l'agent d'imagerie étant en particulier un colorant.

9. Procédé selon l'une quelconque des revendications précédentes, dans lequel l'étape consistant à injecter est réalisée sous agitation.

10. Procédé selon l'une quelconque des revendications précédentes, dans lequel l'agent de ciblage est au moins un peptide choisi parmi la liste suivante avec un degré d'identité d'au moins 90% par rapport à la séquence suivante : SEQ- ID. NO 1, SEQ- ID. NO 2, SEQ- ID. NO 3, ou des mélanges de ceux-ci.

11. Procédé selon l'une quelconque des revendications précédentes, dans lequel la phase aqueuse est une saline tamponnée au phosphate, PBS.

12. Procédé selon l'une quelconque des revendications précédentes dans lequel la phase éthanolique comprend des phospholipides anioniques, neutres ou cationiques, les phospholipides de phase éthanolique étant préférablement choisis parmi une liste consistant en: phosphatidylcholines, phosphatidyléthanolamines, phosphatidylsérines, phosphatidylglycérols et/ou des mélanges de ceux-ci, en particulier de la 1,2-dioléoyl-sn-glycéro-3-phosphoéthanolamine.

13. Procédé selon l'une quelconque des revendications précédentes dans lequel la phase éthanolique comprend également un agent furtif, un agent de ciblage, ou des mélanges de ceux-ci.

14. Procédé selon la revendication 13 dans lequel l'agent furtif est du polyéthylène glycol, PEG, ou des gangliosides, le polyéthylène glycol, PEG étant préférablement lié à un phospholipide, en particulier la distéaroylphosphatidyléthanolamine.

15. Procédé selon l'une quelconque des revendications précédentes dans lequel l'agent de ciblage est incorporé dans la membrane lipidique et/ou dans lequel le principe actif est un agent d'imagerie ou thérapeutique.

16. Procédé selon l'une quelconque des revendications précédentes dans lequel le principe actif est un agent d'imagerie ou thérapeutique qui est hydrophobe ou hydrophile ; préférablement dans lequel l'agent d'imagerie est un colorant ou dans lequel le principe actif est le méthotrexate, la doxorubicine et/ou des mélanges de ceux-ci.
